# EUROPEAN PATENT SPECIFICATION

(11) **EP 2 438 445 B1**
(45) Date of publication and mention of the grant of the patent: **16.07.2014**
(21) Application number: 10724454.3
(22) Date of filing: 02.06.2010
(51) Int. Cl.: G01N 33/574

(54) **METHODS FOR DIAGNOSING PROSTATE CARCINOMAS**
VERFAHREN ZUR DIAGNOSE VON PROSTATAKARZINOMEN
PROCÉDÉS DE DIAGNOSTIC DE CARCINOMES DE PROSTATE

(30) Priority: 04.06.2009 EP 09161956
(43) Date of publication of application: 11.04.2012
(60) Divisional application: 14170939.4
(73) Proprietor: metanomics Health GmbH, 10589 Berlin (DE); Charité Universitätsmedizin Berlin, 10117 Berlin (DE)
(72) Inventor: KAMLAGE, Beate, 12161 Berlin (DE); BETHAN, Bianca, 10717 Berlin (DE); RESZKA, Regina, 16341 Panketal (DE); LEIBOLD, Edgar, 67316 Carlsberg (DE); JUNG, Klaus, 10409 Berlin (DE); LEIN, Michael, 14532 Stahnsdorf (DE); KRISTIANSEN, Glen, CH-8044 Zürich (CH)
(74) Representative: Dick, Alexander
(86) International application number: PCT/EP2010/057680
(87) International publication number: WO 2010/139711

(56) References cited:
- WO-A1-2007/109881
- US-A1- 2009 075 284
- OSL M ET AL: "A new rule-based algorithm for identifying metabolic markers in prostate cancer using tandem mass spectrometry" BIOINFORMATICS, vol. 24, no. 24, December 2008 (2008-12), pages 2908-2914, XP002592499
- SHUKER D E G ET AL: "RELEVANCE OF URINARY DNA ADDUCTS AS MARKERS OF CARCINOGEN EXPOSURE" CHEM RES TOXICOL, vol. 5, no. 4, 1992, pages 450-460, XP002592500
- PORCELLI B ET AL: "Fast-atom bombardment mass spectrometry for mapping of endogenous methylated purine bases in urine extracts" RAPID COMMUN MASS SP, vol. 11, no. 4, 1997, pages 398-404, XP002592501

## Description

The present invention relates to an ex vivo method, for diagnosing prostate carcinomas or predisposition thereof comprising determining at least one metabolite in a test sample of a subject suspected to suffer from prostate carcinomas or to have a predisposition therefore and comparing said at least one metabolite to a reference, whereby prostate carcinomas or a predisposition therefor is to be diagnosed. Moreover, the present invention encompasses the use of said at least one metabolite for diagnosing prostate carcinomas.

Prostate cancer (PCA) is an uncontrolled (malignant) growth of cells in the prostate gland and the most common malignancy in the western world. The cause of prostate cancer isn't fully understood at present. More than 180,000 new cases of PCA patients were diagnosed 2008 in the U.S. The expected prevalence for the US will be 2,175,699 prostate cancer patients in 2010. There are several tests used to diagnose prostate cancer which include Digital rectal examination (DRE), Transrectal ultrasound (TRUS), Prostate-specific antigen (PSA) free and total" PSA derivatives as percent free PSA (%fPSA), age-specific PSA values, PSA density, PSA velocity, Prostatic acid phosphatase (PAP) test, Prostate biopsy, Computed tomography (CT scan), Bone scan and/or MRI.

The prostate-specific antigen -PSA- plasma test has been a major factor in increasing awareness and better patient management of PCA, but its lack of specificity limits its use in diagnosis and makes it suitable for early detection of PCA. PSA is organ but not cancer specific. This leads to a high number of false-positive findings. The diagnosis of PCA can be confirmed only by a biopsy. More than 800,000 of this invasive and costly procedure were done every year in the U.S. Although controversies over PCA screening persist (Wilson SS, Crawford ED 2004, Clin. Prostate Cancer 3: 21-25, Crawford ED 2005, Lancet 365: 1447-1449). The early detection of PCA is likely (Schröder FH et al, 2009; N Engl J Med. Mar 26;360(13):1320-1328; Andriole GL et al . 2009 N Engl J Med Mar 26;360(13):1310-1319 to result in an intervention at an earlier stage of disease and, thus, an increased treatment success. But early detection of PCA and disease progression are lacking for definitive markers for the disease.

The use of metabolomics to generate biomarkers for cancer diagnosis or prognosis and therapeutic evaluation is currently in focus (Spratlin 2009, Clin Cancer Res 15(2): 431-440). Serkova et al 2008;(The Prostate 68: 620-628) had published absolute concentrations of the metabolites citrate, myo-inositol and spermine in human expressed prostatic secretions (EPS) as age-independent markers of PCA. Recently, metabolomic profiles from tissue plasma and post-digital-rectal-exam urine samples of biopsy-positive PCA patients and biopsy-negative control individuals were assayed (Sreekumar 2009, Nature 457(12): 910-914). Sarcosine was suggested as one potential biomarker for prostate cancer progression.

Osl et al 2008, Bioinfomratics 24(24): 2908-2914, US 2009/0075284 A1 and WO 2007/109881 A1 disclose further metabolic markers for prostate cancer. Porcelli et al 1997, Rapid Commun Mass Spectrom 11: 398-404 disclose the use of the metabolite 7 - methylguanine as biomarker for the diagnosis of colorectal carcinoma.

Nevertheless, in light of the high number of false-positive or false-negative diagnosis of prostate carcinoma, there is still a high need for biomarkers which allow for a reliable and efficient diagnosis or prognosis of prostate carcinoma.

Accordingly, the present invention relates to a method for diagnosing a prostate carcinoma or a predisposition therefor comprising:
(a) determining the metabolite 7-Methylguanine and optionally at least one additional metabolite in a test sample of a subject suspected to suffer from a prostate carcinoma or to have a predisposition therefor, said at least one additional metabolite being selected from the group consisting of: 2-Hydroxybehenic acid (C22:0), Cerebronic acid (2-OH-C24:0), Isopentenyl pyrophosphate (IPP), 14-Methylhexadecanoic acid, 2-Aminoadipinic acid, Ceramide (d18:1, C24:1), Eicosenoic acid (C20:cis[11]1), Tricosanoic acid (C23:0), Glycerophosphoethanolamine, Eicosadienoic acid (C20:2) No 02 as defined in Table 5, Arginine, Behenic acid (C22:0), beta-Carotene, Cholestenol No 02 as defined in Table 5, Cytosine, DAG (C18:1, C18:2), Dihydrocholesterol, Erythro-Dihydrosphingosine, Docosahexaenoic acid (C22:cis[4,7,10,13,16,19]6), Dodecanol, Eicosanoic acid (C20:0), Eicosapentaenoic acid (C20:cis[5,8,11,14,17]5), Dihomo-gamma-Linolenic acid (C20:cis[8,11,14]3), erythro-C16-Sphingosine, Flavine adenine dinucleotide (FAD), gamma-Tocopherol, Gluconic acid, Glucuronic acid, Glycerol-2-phosphate, Lignoceric acid (C24:0), Lysophosphatidylcholine (C18:2), Lysophosphatidylcholine (C20:4), Maltotriose, myo-Inositol, myo-Inositol-2-phosphate, Nervonic acid (C24:cis[15]1), Nicotinamide, Pentadecanol, Phosphatidylcholine (C18:0, C22:6), Phytosphingosine, Pseudouridine, Pyruvate, 3-O-Methylsphingosine, threo-Sphingosine, 5-O-Methylsphingosine, erythro-Sphingosine, Sphingosine-1-phosphate, Threonic acid, Sphingosine Isomer No 01 as defined in Table 5, Choline plasmalogen (C18,C20:4), 2-Oxoisocaproic acid, MetID(68300015) as defined in Table 5, MetID(68300047) as defined in Table 5, Erythronic acid, myo-Inositolphospholipids, MetID(38300600) as defined in Table 5, 1,5-Anhydrosorbitol, 3-Hydroxybutyrate, 3-Methoxytyrosine, 4-Hydroxy-3-methoxyphenylglycol (HMPG), 5-Hydroxy-3-indoleacetic acid (5-HIAA), beta-Sitosterol, Canthaxanthin, Ceramide (d18:1, C24:0), MetID(68300017) as defined in Table 5, Coenzyme Q10, conjugated Linoleic acid (C18:trans[9,11]2), Cryptoxanthin, Dihydrotestosterone, gamma-Linolenic acid - (C18:cis[6,9,12]3), Glycerate, Lactate, Lycopene, Lysophosphatidylcholine (16:0), Lysophosphatidylcholine (C17:0), Phosphatidylcholine (C16:0, C20:4), Phosphatidylcholine (C18:0, C18:2), Phosphatidylcholine (C18:2, C20:4), MetID(68300048) as defined in Table 5, Phosphatidylcholine, MetID(68300020) as defined in Table 5, scyllo-Inositol, Sphingomyelin (d18:1, C16:0), MetID(68300045) as defined in Table 5, Testosterone, and Dehydroepiandrosterone sulfate ; and
(b) comparing the results of the determination in step (a) to a reference, whereby said prostate carcinoma or a predisposition therefor is to be diagnosed.

7-Methylguanine is a suitable biomarker by its own for the diseases referred to herein. However, most preferably, a group of biomarkers is to be determined by the method of the present invention. A group of biomarkers comprises or consists, preferably, of at least two, at least three, at least four and, preferably, up to all of the aforementioned biomarkers.

More preferably, the said at least one additional metabolite is selected from the group consisting of: 2-Hydroxybehenic acid (C22:0), Cerebronic acid (2-OH-C24:0), Isopentenyl pyrophosphate (IPP), 14-Methylhexadecanoic acid, 2-Aminoadipinic acid, Ceramide (d18:1, C24:1), Ceramide (d18:2, C24:0), Eicosenoic acid (C20:cis[11]1), Tricosanoic acid (C23:0), Glycerophosphoethanolamine, Eicosadienoic acid (C20:2) No 02 as defined in Table 5.

The expression "method for diagnosing" as referred to in accordance with the present invention means that the method may essentially consist of the aforementioned steps or may include further steps. However, it is to be understood that the method, is a method carried out in vitro, i.e. not practised on the human or animal body. Diagnosing as used herein refers to assessing the probability according to which a subject is suffering from a disease. As will be understood by those skilled in the art, such an assessment, although preferred to be, may usually not be correct for 100% of the subjects to be diagnosed. The term, however, requires that a statistically significant portion of subjects can be identified as suffering from the disease or as having a predisposition therefor. Whether a portion is statistical significant can be determined without further ado by the person skilled in the art using various well known statistic evaluation tools, e.g., determination of confidence intervals, p-value determination, Student's t-test, Mann-Whitney test, etc.. Details are found in Dowdy and Wearden, Statistics for Research, John Wiley & Sons, New York 1983. Preferred confidence intervals are at least 50%, at least 60%, at least 70%, at least 80%, at least 90%, at least 95%. The p-values are, preferably, 0.2, 0.1, 0.05. It will be understood, moreover, that the methods of the present invention essentially provide an aid for diagnosis and may be included into or supplemented by other diagnostic measures.

Diagnosing according to the present invention includes monitoring, confirmation, and classification of the relevant disease or its symptoms. Monitoring relates to keeping track of an already diagnosed disease, or a complication, e.g. to analyze the progression or regression of the disease, the influence of a particular treatment on the progression of disease or complications arising during the disease period or after successful treatment of the disease. Confirmation relates to the strengthening or substantiating a diagnosis already performed using other indicators or markers. Classification relates to allocating the diagnosis according to the strength or kind of symptoms into different classes, e.g. the stages for prostate carcinomas as set forth elsewhere in the description.

The term "prostate carcinoma" as used herein refers to a malignant tumor developed from prostate gland cells. Said cells may metastasize from the prostate to other tissues or organs, especially to the bones and lymph nodes. Early prostate carcinomas usually cause no symptoms. Prostate carcinomas in advanced stages may cause pain, difficulty in urinating, problems during sexual intercourse, and erectile dysfunction. Further symptoms of prostate carcinomas are well known in the art including frequent urination, increased urination at night, difficulty starting and maintaining a steady stream of urine, blood in the urine, and painful urination and are described in the standard text books of medicine, such as Stedman or Pschyrembl. Prostate carcinomas are classified The Gleason scoring system is used to grade prostate tumors from 2 to 10, whereby a Gleason score of 10 indicates the most abnormalities. Proper staging and grading of the tumor is important in order to allow for efficient and appropriate therapeutic interventions.

The term "predisposition" as used herein means that a subject has not yet developed the disease or any of the aforementioned disease symptoms or other diagnostic criteria but, nevertheless, will develop the disease in the future with a certain likelihood. Said likelihood shall differ significantly from the likelihood of statistical appearance of prostate carcinomas. Preferably, the likelihood for developing a prostate carcinoma is at least 30%, at least 40%, at least 50%, at least 60%, at least 70%, at least 80%, at least 90% or 100% of a predisposition is diagnosed. Diagnosis of a predisposition may sometimes be referred to as prediction of the likelihood that a subject will develop the disease.

The term "at least one metabolite" as used herein refers to a single metabolite or to a plurality of metabolites, i.e. preferably at least 2, 3, 4, 5, 10, 50, 100, 500, 1,000, 2,000, 3,000, 5,000 or 10,000 metabolites. It is to be understood that "metabolite" as used herein may be at least one molecule of said metabolite up to a plurality of molecules of the metabolite and that a plurality of metabolites means a plurality of chemically different molecules wherein for each metabolite at least one molecule up to a plurality of molecules may be present. A metabolite in accordance with the present invention encompasses all classes of organic or inorganic chemical compounds including those being comprised by biological material such as organisms. Preferably, the metabolite in accordance with the present invention is a small molecule compound. More preferably, in case a plurality of metabolites is envisaged, said plurality of metabolites representing a metabolome, i.e. the collection of metabolites being comprised by an organism, an organ, a tissue or a cell at a specific time and under specific conditions.

The metabolites are small molecule compounds, such as substrates for enzymes of metabolic pathways, intermediates of such pathways or the products obtained by a metabolic pathway. Metabolic pathways are well known in the art and may vary between species. Preferably, said pathways include at least citric acid cycle, respiratory chain, photosynthesis, photorespiration, glycolysis, gluconeogenesis, hexose monophosphate pathway, oxidative pentose phosphate pathway, production and β-oxidation of fatty acids, urea cycle, amino acid biosynthesis pathways, protein degradation pathways such as proteasomal degradation, amino acid degrading pathways, biosynthesis or degradation of: lipids, polyketides (including e.g. flavonoids and isoflavonoids), isoprenoids (including eg. terpenes, sterols, steroids, carotenoids, xanthophylls), carbohydrates, phenylpropanoids and derivatives, alcaloids, benzenoids, indoles, indole-sulfur compounds, porphyrines, anthocyans, hormones, vitamins, cofactors such as prosthetic groups or electron carriers, lignin, glucosinolates, purines, pyrimidines, nucleosides, nucleotides and related molecules such as tRNAs, microRNAs (miRNA) or mRNAs. Accordingly, small molecule compound metabolites are preferably composed of the following classes of compounds: alcohols, alkanes, alkenes, alkines, aromatic compounds, ketones, aldehydes, carboxylic acids, esters, amines, imines, amides, cyanides, amino acids, peptides, thiols, thioesters, phosphate esters, sulfate esters, thioethers, sulfoxides, ethers, or combinations or derivatives of the aforementioned compounds. The small molecules among the metabolites may be primary metabolites which are required for normal cellular function, organ function or animal growth, development or health. Moreover, small molecule metabolites further comprise secondary metabolites having essential ecological function, e.g. metabolites which allow an organism to adapt to its environment. Furthermore, metabolites are not limited to said primary and secondary metabolites and further encompass artificial small molecule compounds. Said artificial small molecule compounds are derived from exogenously provided small molecules which are administered or taken up by an organism but are not primary or secondary metabolites as defined above. For instance, artificial small molecule compounds may be metabolic products obtained from drugs by metabolic pathways of the animal. Moreover, metabolites further include peptides, oligopeptides, polypeptides, oligonucleotides and polynucleotides, such as RNA or DNA. More preferably, a metabolite has a molecular weight of 50 Da (Dalton) to 30,000 Da, most preferably less than 30,000 Da, less than 20,000 Da, less than 15,000 Da, less than 10,000 Da, less than 8,000 Da, less than 7,000 Da, less than 6,000 Da, less than 5,000 Da, less than 4,000 Da, less than 3,000 Da, less than 2,000 Da, less than 1,000 Da, less than 500 Da, less than 300 Da, less than 200 Da, less than 100 Da. Preferably, a metabolite has, however, a molecular weight of at least 50 Da. Most preferably, a metabolite in accordance with-the present invention has a molecular weight of 50 Da up to 1,500 Da.

It will be understood that in addition to the aforementioned metabolites or groups of metabolites, an additional biomarker or a group of additional biomarkers can be determined by the method of the present invention as well. Said additional biomarkers include nucleic acids, peptides, polypeptides or other clinical parameters known to be associated with prostate carcinomas or predisposition therefore. Preferably, said additional biomarker is selected from the group consisting of: Digital rectal examination (DRE), Transrectal ultrasound (TRUS), PSA and PAP Tests, Prostate-specific antigen (PSA), Free and total PSA (also known as PSA II), Age-specific PSA, Prostatic acid phosphatase (PAP) test Tumor Biopsy, Computed tomography (CT scan), Bone scan and MRI.

A preferred metabolite to be determined as biomarker together, i.e. either simultaneously or consecutively, with the aforementioned metabolites or groups of metabolites is at least one metabolite selected from the group consisting of: Biotin, Uridine, Hypoxanthine, Inosine, Glycine, Cysteine, Cystine, Uracil, Aspartate, Isoleucine, trans-4-Hydroxyproline, Proline, Methionine, Glycerol-3-phosphate, 5-Oxoproline, Folic acid, Glutamate, Glutamine, Leucine, Myristic acid (C14:0), Phenylalanine, Heptadecanoic acid (C17:0), Citrulline, Threonine, myo-Inositol-1-phosphate, myo-Inositolphospholipids, Ribose, Fumarate, Tryptophan, Glycerol, Tyrosine, Homoserine, Histidine, Linoleic acid (C18:cis[9,12]2), Xanthine, Ornithine, Arginine, Citrulline, Pantothenic acid, Palmitoleic acid (C16:cis[9]1), Succinate, Fructose, alpha-Tocopherol, Nicotineamide adenine dinucleotide (NAD), Maltose, Valine, Adenine, Lysine, Malate, Alanine, Spermidine, Palmitic acid (C16:0), Stearic acid (C18:0), Oleic acid (C18:cis[9]1), Glycerol phosphate, N-Acetylneuraminic acid, Xylitol, Serine, N-Acetylneuraminic acid, S-Adenosylmethionine, Phosphate, Glucose, Cholesterol, Spermine, Putrescine, cis-Aconitate, Citrate, Ribulose-5-phosphate, Pyrophosphate (PPi), Elaidic acid (C18:trans[911), Adenine, 2-Hydroxybutyrate, Sarcosine, Isocitrate, Creatine, Glutathione disulfide (GSSG), 3-Hydroxybutyrate, and Taurine.

The supportive metabolites referred to before will, preferably, also be compared to suitable reference results as specified elsewhere herein. The result of the said comparison will be further supportive for the finding as to whether the subject will suffer from prostate carcinomas or not or will have a predisposition therefor or not. Preferred reference results, values for changes of the relative amounts and indications for the kind of regulation are to be found in the accompanying Examples, below.

The term "test sample" as used herein refers to samples to be used for the diagnosis of prostate carcinomas or a predisposition therefor by the method of the present invention. Said test sample is a biological sample. Samples from biological sources (i.e. biological samples) usually comprise a plurality of metabolites. Preferred biological samples to be used in the method of the present invention are samples from body fluids, preferably, blood, plasma, serum, lymph, or urine, or samples derived, e.g., by biopsy, from cells, tissues or organs, preferably prostate tissue suspected to include or essentially consist of prostate carcinoma cells. This also encompasses samples comprising subcellular compartments or organelles, such as the mitochondria, Golgi network or peroxisomes. Biological samples can be derived from a subject as specified elsewhere herein. Techniques for obtaining the aforementioned different types of biological samples are well known in the art. For example, blood samples may be obtained by blood taking while tissue or organ samples are to be obtained, e.g., by biopsy.

The aforementioned samples are, preferably, pre-treated before they are used for the method of the present invention. As described in more detail below, said pre-treatment may include treatments required to release or separate the compounds or to remove excessive material or waste. Suitable techniques comprise centrifugation, extraction, fractioning, purification and/or enrichment of compounds. Moreover, other pre-treatments are carried out in order to provide the compounds in a form or concentration suitable for compound analysis. For example, if gas-chromatography coupled mass spectrometry is used in the method of the present invention, it will be required to derivatize the compounds prior to the said gas chromatography. Suitable and necessary pre-treatments depend on the means used for carrying out the method of the invention and are well known to the person skilled in the art. Pre-treated samples as described before are also comprised by the term "sample" as used in accordance with the present invention.

The term "subject" as used herein relates to animals, preferably to mammals such as mice, rats, sheep, dogs, cats, horses, monkeys, or cows and, also preferably, to humans. Other animals which may be diagnosed applying the method of the present invention are birds or reptiles. A subject suspected to suffer from prostate carcinoma or to have a predisposition therefor as used herein refers to a subject which shows, preferably, symptoms or clinical signs or parameters indicative for prostate carcinomas. However, the term also relates to an apparently healthy subject, i.e. a subject not exhibiting any of the aforementioned symptoms, clinical signs or parameters. Apparently healthy subjects may by investigated by the method of the present invention as a measure of preventive care or for population screening purposes.

The term "determining said at least one metabolite" as used herein refers to determining at least one characteristic feature of the at least one metabolite comprised by the sample referred to herein. Characteristic features in accordance with the present invention are features which characterize the physical and/or chemical properties including biochemical properties of a metabolite. Such properties include, e.g., molecular weight, viscosity, density, electrical charge, spin, optical activity, colour, fluorescence, chemoluminescence, elementary composition, chemical structure, capability to react with other compounds, capability to elicit a response in a biological read out system (e.g., induction of a reporter gene) and the like. Values for said properties may serve as characteristic features and can-be determined by techniques well known in the art. Moreover, the characteristic feature may be any feature which is derived from the values of the physical and/or chemical properties of a metabolite by standard operations, e.g., mathematical calculations such as multiplication, division or logarithmic calculus. Most preferably, the at least one characteristic feature allows the determination and/or chemical identification of the said at least one metabolite.

The at least one metabolite comprised by a test sample may be determined in accordance with the present invention quantitatively or qualitatively. For qualitative determination, the presence or absence of the metabolite will be determined by a suitable technique. Moreover, qualitative determination may, preferably, include determination of the chemical structure or composition of the metabolite. For quantitative determination, either the precise amount of the at least one metabolite present in the sample will be determined or the relative amount of the at least one metabolite will be determined, preferably, based on the value determined for the characteristic feature(s) referred to herein above. The relative amount may be determined in a case were the precise amount of a metabolite can or shall not be determined. In said case, it can be determined whether the amount in which the metabolite is present is enlarged or diminished with respect to a second sample comprising said metabolite in a second amount. Quantitatively analysing a metabolite, thus, also includes what is sometimes referred to as semi-quantitative analysis of a metabolite.

Moreover, determining as used in the method according to the present invention, preferably, includes using a compound separation step prior to the analysis step referred to before. Preferably, said compound separation step yields a time resolved separation of the metabolites comprised by the sample. Suitable techniques for separation to be used preferably in accordance with the present invention, therefore, include all chromatographic separation techniques such as liquid chromatography (LC), high performance liquid chromatography (HPLC), gas chromatography (GC), thin layer chromatography, size exclusion or affinity chromatography. These techniques are well known in the art and can be applied by the person skilled in the art without further ado. Most preferably, LC and/or GC are chromatographic techniques to be envisaged by the method of the present invention. Suitable devices for such determination of metabolites are well known in the art. Preferably, mass spectrometry is used in particular gas chromatography mass spectrometry (GC-MS), liquid chromatography mass spectrometry (LC-MS), direct infusion mass spectrometry or Fourier transform ion-cyclotrone-resonance mass spectrometry (FT-ICR-MS), capillary electrophoresis mass spectrometry (CE-MS), high-performance liquid chromatography coupled mass spectrometry (HPLC-MS), quadrupole mass spectrometry, any sequentially coupled mass spectrometry, such as MS-MS or MS-MS-MS, inductively coupled plasma mass spectrometry (ICP-MS), pyrolysis mass spectrometry (Py-MS), ion mobility mass spectrometry or time of flight mass spectrometry (TOF). Most preferably, LC-MS and/or GC-MS are used as described in detail below. Said techniques are disclosed in, e.g., Niessen, Journal of Chromatography A, 703, 1995: 37-57, US 4,540,884 or US 5,397,894. As an alternative or in addition to mass spectrometry techniques, the following-techniques may be used for compound determination: nuclear magnetic resonance (NMR), magnetic resonance imaging (MRI), Fourier transform infrared analysis (FT-IR), ultra violet (UV) spectroscopy, refraction index (RI), fluorescent detection, radiochemical detection, electrochemical detection, light scattering (LS), dispersive Raman spectroscopy or flame ionisation detection (FID). These techniques are well known to the person skilled in the art and can be applied without further ado. The method of the present invention shall be, preferably, assisted by automation. For example, sample processing or pre-treatment can be automated by robotics. Data processing and comparison is, preferably, assisted by suitable computer programs and databases. Automation as described herein before allows using the method of the present invention in high-throughput approaches.

Moreover, the at least one metabolite can also be determined by a specific chemical or biological assay. Said assay shall comprise means which allow to specifically detect the at least one metabolite in the sample. Preferably, said means are capable of specifically recognizing the chemical structure of the metabolite or are capable of specifically identifying the metabolite based on its capability to react with other compounds or its capability to elicit a response in a biological read out system (e.g., induction of a reporter gene). Means which are capable of specifically recognizing the chemical structure of a metabolite are, preferably, antibodies or other proteins which specifically interact with chemical structures, such as receptors or enzymes. Specific antibodies, for instance, may be obtained using the metabolite as antigen by methods well known in the art. Antibodies as referred to herein include both polyclonal and monoclonal antibodies, as well as fragments thereof, such as Fv, Fab and F(ab)₂ fragments that are capable of binding the antigen or hapten. They also include humanized hybrid antibodies wherein amino acid sequences of a non-human donor antibody exhibiting a desired antigen-specificity are combined with sequences of a human acceptor antibody. Moreover, encompassed are single chain antibodies. The donor sequences will usually include at least the antigen-binding amino acid residues of the donor but may comprise other structurally and/or functionally relevant amino acid residues of the donor antibody as well. Such hybrids can be prepared by several methods well known in the art. Suitable proteins which are capable of specifically recognizing the metabolite are, preferably, enzymes which are involved in the metabolic conversion of the said metabolite. Said enzymes may either use the metabolite as a substrate or may convert a substrate into the metabolite. Moreover, said antibodies may be used as a basis to generate oligopeptides which specifically recognize the metabolite. These oligopeptides shall, for example, comprise the enzyme's binding domains or pockets for the said metabolite. Suitable antibody and/or enzyme based assays may be RIA (radioimmunoassay), ELISA (enzyme-linked immunosorbent assay), sandwich enzyme immune tests, electrochemiluminescence sandwich immunoassays (ECLIA), dissociation-enhanced lanthanide fluoro immuno assay (DELFIA) or solid phase immune tests. Moreover, the metabolite may also be identified based on its capability to react with other compounds, i.e. by a specific chemical reaction. Suitable reactions are well known in the art and, preferably encompass enzymatic reactions (e.g for mannose Pitkanen E, Pitkanen O, Uotila L.; Eur J Clin Chem Clin Biochem. 1997 Oct;35(10):761-6; or ascorbic acid Winnie Lee, Susan M. Roberts and Robert F. Labbe; Clinical Chemistry 43: 154-157, 1997), enzymatic spectrophotometric methods (BN La Du, RR Howell, PJ Michael and EK Sober; Pediatrics, Jan 1963, 39-46, Vol 31, No. 1), spectrofluorimetric methods (Sumi T, Umeda Y, Kishi Y, Takahashi K, Kaki-moto F.; Clin Chim Acta. 1976 Dec 1;73(2):233-9) and fluorescence; chemiluminescence (J.J. Thiele, H.J. Freisleben, J. Fuchs and F.R. Ochsendorf; Human Reproduction, Vol. 10, No. 1, pp. 110-115, 1995). Further detection methods such as capillary electrophoresis (Hubert A. Carchon and Jaak Jaeken; Clinical Chemistry 47: 1319-1321, 2001) and colorimetric methods (Kyaw A; Clin Chim Acta. 1978 Jun;86(2):153-7) can be used. Further, the metabolite may be determined in a sample due to its capability to elicit a response in a biological read out system. The biological response shall be detected as read out indicating the presence and/or the amount of the metabolite comprised by the sample. The biological response may be, e.g., the induction of gene expression or a phenotypic response of a cell or an organism.

Further, it is to be understood that depending of the technique used for determining the said at least one metabolite, the analyte which will be detected could be a derivative of the physiologically occurring metabolite, i.e. the metabolite present within a subject. Such analytes may be generated as a result of sample preparation or detection means. The compounds referred to herein are deemed to be analytes. However, as set forth above, these analytes will represent the metabolites in a qualitative and quantitative manner. Moreover, it is to be understood that for a plurality of metabolites, the metabolite will be identical to the analyte.

A metabolite or analyte as referred to in accordance with the present invention refers to a molecular species which serves as an indicator for a disease or effect as referred to in this specification. Said molecular species can be a metabolite itself which is found in a sample of a subject. Moreover, the biomarker may also be a molecular species which is derived from said metabolite. In such a case, the actual metabolite will be chemically modified in the sample or during the determination process and, as a result of said modification, a chemically different molecular species, i.e. the analyte, will be the determined molecular species. It is to be understood that in such a case, the analyte represents the actual metabolite and has the same potential as an indicator for the respective medical condition. Moreover, a biomarker according to the present invention is not necessary corresponding to one molecular species. Rather, the biomarker may comprise stereoisomers or enantiomeres of a compound. Further, a biomarker can also represent the sum of isomers of a biological class of isomeric molecules. Said isomers shall exhibit identical analytical characteristics in some cases and are, therefore, not distinguishable by various analytical methods including those applied in the accompanying Examples described below. However, the isomers will share at least identical sum formula parameters and, thus, in the case of, e.g., lipids an identical chain-length and identical numbers of double bonds in the fatty acid and/or sphingo base moieties.

The term "reference" refers to results, i.e. data of characteristic features of the at least one metabolite, which can be correlated to prostate carcinoma or a predisposition therefor. Such reference results are, preferably, obtained from a sample from a subject known to suffer from prostate carcinomas or a subject known to have predisposition therefor. The reference can also be the average or mean obtained from a group of such samples. If the reference shall be obtained from a biopsy tissue sample, the said sample shall comprise or essentially consists of prostate carcinoma tissue. The reference results may be obtained by applying the method of the present invention. Alternatively, but nevertheless also preferred, the reference results may be obtained from sample of a subject known not to suffer from prostate carcinomas or a subject known not to have a predisposition therefore, i.e. a healthy subject with respect to prostate carcinomas and, more preferably, other diseases, in particular cancer diseases, as well. Likewise, if the reference shall be obtained from a biopsy tissue sample, the said sample shall essentially consists of apparently healthy prostate tissue. The reference can also be the average or mean obtained from a group of such samples. Preferably, if biopsy tissues samples are envisaged, the sample underlying the reference and the test sample can be obtained from the same subject, i.e. from areas which are apparently affected by prostate carcinoma and from areas suspected to be affected by prostate carcinoma. Moreover, the reference, also preferably, could be a calculated reference, most preferably the average or median, for the relative or absolute amount of a metabolite of a representative population of individuals which are apparently healthy or suffer from prostate carcinoma, wherein the subjects suffering from prostate carcinoma are within the prevalence for the disease in a given population, preferably, the US, Asian or European population The absolute or relative amounts of the metabolites of said individuals of the population can be determined as specified elsewhere herein. How to calculate a suitable reference value, preferably, the average or median, is well known in the art. The population of subjects referred to before shall comprise a plurality of subjects, preferably, at least 5, 10, 50, 100, 1,000 or 10,000 subjects. It is to be understood that the subject to be diagnosed by the method of the present invention and the subjects of the said plurality of subjects are of the same species.

More preferably, a "reference" will be obtained by determining the values for the at least one characteristic feature for a group of reference subjects, i.e. a group of subjects known to suffer from prostate carcinoma, a group of subjects known not to suffer from prostate carcinoma, a population comprising the subject to be investigated or a group of tissue biopsy samples of prostate carcinoma tissue or apparently healthy tissue and calculating the reference by appropriate statistic measures including those referred to elsewhere herein, such as median, average, quantiles, PLS-DA, logistic regression methods, random forest classification or others that give a threshold value. The threshold value should take the desired clinical settings of sensitivity and specificity of the diagnostic and prognostic test into consideration.

More preferably, the reference results, i.e. values for at least one characteristic features of the at least one metabolite, will be stored in a suitable data storage-medium such as a database and are, thus, also available for future diagnoses. This also allows efficiently diagnosing predisposition for a disease because suitable reference results can be identified in the database once it has been confirmed (in the future) that the subject from which the corresponding reference sample was obtained (indeed) developed prostate carcinoma. Preferred reference results which are associated with prostate carcinoma or predisposition therefore in humans are those shown in the Tables of the accompanying Examples.

The term "comparing" refers to assessing whether the results of the determination described hereinabove in detail, i.e. the results of the qualitative or quantitative determination of the at least one metabolite, are identical or similar to reference results or differ therefrom.

In case the reference results are obtained from a subject or a group known to suffer from prostate carcinomas or known to have a predisposition for prostate carcinomas or from a tissue sample comprising or essentially consisting of prostate carcinoma, the said disease or predisposition can be diagnosed based on the degree of identity or similarity between the test results-obtained from the test sample and the aforementioned reference results, i.e. based on an identical or similar qualitative or quantitative composition with respect to the at least one metabolite. The results of the test sample and the reference results are identical, if the values for the characteristic features and, in the case of quantitative determination, the intensity values are identical. Said results are similar, if the values of the characteristic features are identical but the intensity values are different. Such a difference is, preferably, not significant and shall be characterized in that the values for the intensity are within at least the interval between 1^{st} and 99^{th} percentile, 5^{th} and 95^{th} percentile, 10^{th} and 90^{th} percentile, 20^{th} and 80^{th} percentile, 30^{th} and 70^{th} percentile, 40^{th} and 60^{th} percentile of the reference value or the 50^{th}, 60^{th}, 70^{th}, 80^{th}, 90^{th} or 95^{th} percentile of the reference value.

In case the reference results are obtained from a subject or a group known not to suffer from prostate carcinomas or known not to have a predisposition for prostate carcinomas or from a tissue sample-essentially consisting of apparently healthy prostate tissue, the said disease or predisposition can be diagnosed based on the differences between the test results obtained from the test sample and the aforementioned reference results, i.e. differences in the qualitative or quantitative composition with respect to the at least one metabolite. The same applies if a calculated reference as specified above is used. The difference may be an increase in the absolute or relative amount of a metabolite (sometimes referred to as up-regulation of the metabolite; see also Examples) or a decrease in either of said amounts or the absence of a detectable amount of the metabolite (sometimes referred to as down-regulation of the metabolite; see also Examples). Preferably, the difference in the relative or absolute amount is significant, i.e. outside of the interval between 45^{th} and 55^{th} percentile, 40^{th} and percentile, 30^{th} and 70^{th} percentile and 80^{th} percentile, 10^{th} and 90^{th} percentile, 5^{th} and 95^{th} percentile, 1^{st} and 99^{th} percentile of the reference value.

For the specific metabolites referred to in this specification elsewhere, preferred values for the changes in the relative amounts (i.e. changes in the median) or the kind of regulation (i.e. "up"- or "down"-regulation resulting in a higher or lower relative and/or absolute amount) are indicated in the Tables below. If if is indicated in said tables that a given metabolite is "up- regulated" in a subject or a tissue sample, the relative and/or absolute amount will be increased, if it is "down-regulated", the relative and/or absolute amount of the metabolite will be decreased. Moreover, the Median indicates the degree of increase or decrease, e.g., a Median of 2.0 means that the amount is twice the amount of the metabolite compared to the reference.

Thus, the method of the present invention in a preferred embodiment includes a reference that is derived from a subject or a group known to suffer from prostate carcinomas or a subject or a group known to have predisposition therefore or a biopsy tissue sample comprising or essentially consisting of prostate carcinoma tissue Most preferably, identical or similar results for the test sample and the said reference (i.e. similar relative or absolute amounts of the at least one metabolite) are indicative for prostate carcinomas or a predisposition therefor in that case. In another preferred embodiment of the method of the present invention, the reference is derived from a subject known not to suffer from prostate carcinomas or a subject known not to have predisposition therefore or a biopsy tissue sample essentially consisting of apparently healthy prostate tissue. Further, it, preferably, can be a calculated reference. Most preferably, the absence of the at least one metabolite or an amount which, preferably significantly, differs in the test sample in comparison to the reference sample (i.e. a significant difference in the absolute or relative amount is observed) is indicative for prostate carcinomas or predisposition therefore in such a case.

The comparison is, preferably, assisted by automation. For example, a suitable computer program comprising algorithm for the comparison of two different data sets (e.g., data sets comprising the values of the characteristic feature(s)) may be used. Such computer programs and algorithm are well known in the art. Notwithstanding the above, a comparison can also be carried out manually.

The aforementioned methods for the determination of the at least one metabolite can be implemented into a device. A device as used herein shall comprise at least the aforementioned means. Moreover, the device, preferably, further comprises means for comparison and evaluation of the detected characteristic feature(s) of the at least one metabolite and, also preferably, the determined signal intensity. The means of the device are, preferably, operatively linked to each other. How to link the means in an operating manner will depend on the type of means included into the device. For example, where means for automatically qualitatively or quantitatively determining the metabolite or metabolites are applied, the data obtained by said automatically operating means can be processed by, e.g., a computer program in order to facilitate the diagnosis. Preferably, the means are comprised by a single device in such a case. Said device may accordingly include an analyzing unit for the metabolites and a computer unit for processing the resulting data for the diagnosis. Alternatively, where means such as test stripes are used for determining the metabolites, the means for diagnosing may comprise control stripes or tables allocating the determined result data to result data known to-be accompanied with prostate carcinoma or a predisposition therefor or those being indicative for a healthy subject as discussed above. Preferred devices are those which can be applied without the particular knowledge of a specialized clinician, e.g., test stripes or electronic devices which merely require loading with a sample.

Alternatively, the methods for the determination of the at least one metabolite can be implemented into a system comprising several devices which are, preferably, operatively linked to each other. Specifically, the means must be linked in a manner as to allow carrying out the method of the present invention as described in detail above. Therefore, operatively linked, as used herein, preferably, means functionally linked. Depending on the means to be used for the system, said means may be functionally linked by connecting each mean with the other by means which allow data transport in between said means, e.g., glass fiber cables, and other cables for high throughput data transport. Nevertheless, wireless data transfer between the means is also envisaged, e.g., via LAN (Wireless LAN, W-LAN). A preferred system comprises means for determining metabolites. Means for determining metabolites as used herein, encompass means for separating metabolites, such as chromatographic devices, and means for metabolite determination, such as mass spectrometry devices. Suitable devices have been described in detail above. Preferred means for compound separation to be used in the system include chromatographic devices, more preferably devices for liquid chromatography, HPLC, and/or gas chromatography. Preferred devices for compound determination comprise mass spectrometry devices, more preferably, GC-MS, LC-MS, direct infusion mass spectrometry, FT-ICR-MS, CE-MS, HPLC-MS, quadrupole mass spectrometry, sequentially coupled mass spectrometry (including MS-MS or MS-MS-MS), ICP-MS, Py-MS or TOF. The separation and determination means are, preferably, coupled to each other. Most preferably, LC-MS and/or GC-MS is used in the system as described in detail elsewhere in the specification. Further comprised shall be means for comparing and/or analyzing the results obtained from the means for determination of metabolites. The means for comparing and/or analyzing the results may comprise at least one database and an implemented computer program for comparison of the results.

Advantageously, it has been found in accordance with the present invention that at least one or a group of the aforementioned metabolites will be a suitable biomarker for prostate carcinomas or a predisposition therefor. Applying these metabolites as biomarkers allows a rapid, reliable and cost-effective diagnosis of prostate carcinomas. Moreover, an additional advantage over the techniques available in the prior art is that the method of the present invention allows even the diagnosis of a predisposition for developing prostate carcinoms. Moreover, the method can be assisted by automation as described elsewhere in this description and, thus, allows high-throughput screening for subjects being at risk of suffering from prostate carcinomas. Thereby, the method of the present invention may assist health programs for prostate cancer prevention and can be used to monitor success of therapies or measures for the prevention of prostate carcinomas including therapies and nutritional diets. Moreover, .the metabolites or combinations of metabolites referred to herein can be determined simultaneously in a time and cost effective manner by the metabolic profiling techniques described in this specification.

The explanations and interpretations of the terms made above apply accordingly to the other embodiments specified herein below except as indicated otherwise.

In a preferred embodiment of the above method of the present invention, said at least one additional metabolite in a test sample is selected from the group consisting of the metabolites recited in Table 1, below. More preferably, the said test sample is a tissue biopsy sample. Particular preferred changes or kinds of regulation are also indicated in the Table.

In another preferred embodiment of the method of the present invention, said at least one additional metabolite in a test sample is selected from the group consisting of the metabolites recited in Table 3, below. More preferably, said test sample is whole blood, serum or plasma or a fraction of any of these. Particular preferred changes or kinds of regulation are also indicated in the Table.

The present specification further pertains to a method for diagnosing the progression of prostate carcinoma comprising:
(a) determining at least one metabolite in a test sample of a subject suspected to suffer from progressing prostate carcinoma, said at least one metabolite being selected from the group consisting of: MetID (58300131) as defined in Table 5, Phosphatidylcholine (C18:0, C18:2), Phosphatidylcholine (C16:0, C20:4), Phosphatidylcholine (C18:2, C18:2), 2-Oxoisocaproic acid, Erythronic acid, Choline plasmalogen (C18,C20:4), Choline plasmalogen, Lysophosphatidylcholine (18:0), Phosphatidylcholine (C16:0, C16:0), Phosphatidylcholine, Pyruvate, Phosphoenolpyruvate, beta-Sitosterol, and Coenzyme Q10; and
(b) comparing the test results of the determination in step (a) to a reference, whereby said progressing prostate carcinoma is to be diagnosed.

The terms "progression of prostate carcinoma" and "progressing prostate carcinomas" relate to the conversion of prostate carcinomas from one stage to another, preferably, more advanced stage. Preferably, the progression of prostate carcinomas envisaged by the method is progression according to the Gleason scoring system as already mentioned. As set forth elsewhere herein already, the classification and the determination of progression is important in order to-allow for efficient and appropriate therapeutic interventions. In particular, it is desirable to start therapeutic interventions prior to progression of the prostate carcinomas into stages where metastasis occurs.

It will be understood that a reference to be, preferably, used for the aforementioned method for diagnosing the progression of prostate carcinomas shall be derived from a subject known to suffer from a progressing prostate carcinoma, preferably from prostate carcinoma according to at least Gleason stages 3a or 3b. More preferably, identical or similar results for the test sample and the reference are indicative for a progressing prostate carcinoma in such a case. Alternatively, the reference may be, preferably, derived from a subject known to not suffer from a progressing prostate carcinoma, preferably from a subject suffering from prostate carcinoma according to Gleason stages 2a or 2b. More preferably, the absence of the said at least one metabolite or an amount thereof which differs in the test sample in comparison to the reference sample is indicative for a progressing prostate carcinoma in these cases. It will be understood that the reference can be obtained also from a group of such subjects by measures discussed before, e.g., the median or average of the amount of the biomarker may be determined.

Advantageously, it was found that the above recited metabolites are suitable as biomarkers indicating the progression of prostate carcinomas. Based on the method it is, thus, possible to decide on proper therapeutic interventions. For example, systemic chemotherapy may be avoided where a patient has been diagnosed to suffer from a non-progressing prostate carcinoma which has no metastasising potential yet. On the other hand, systemic chemotherapy shall be applied if a patient has been diagnosed to suffer from a progressing prostate carcinoma.

It will be understood that a regression of prostate carcinoma in a subject can also be diagnosed by the aforementioned method. Specifically, the present specification further contemplates a method for diagnosing the regression of prostate carcinoma comprising:
(a) determining at least one metabolite in a test sample of a subject suspected to suffer from regressing prostate carcinoma, said at least one metabolite being selected from the group consisting of: MetID(58300131) as defined in Table 5, Phosphatidylcholine (C18:0, C18:2), Phosphatidylcholine (C16:0, C20:4), Phosphatidylcholine (C18:2, C18:2), 2-Oxoisocaproic acid, Erythronic acid, Choline plasmalogen (C18,C20:4), Choline plasmalogen, Lysophosphatidylcholine (18:0), Phosphatidylcholine (C16:0, C16:0), Phosphatidylcholine, Pyruvate, Phosphoenolpyruvate, beta-Sitosterol, and Coenzyme Q10; and
(b) comparing the test results of the determination in step (a) to a reference, whereby said regressing prostate carcinoma is to be diagnosed.

The terms "regression of prostate carcinoma" and "regressing prostate carcinomas" relate to the conversion of prostate carcinomas from a more advanced stage to a less advanced stage.

It will be understood that a reference to be, preferably, used for the aforementioned method for diagnosing the regression of prostate carcinomas shall be derived from a subject known to suffer from a regressing prostate carcinoma, preferably from prostate carcinoma according to at least Gleason stages 2a or 2b. More preferably, identical or similar results for the test sample and the reference are indicative for a regressing prostate carcinoma in such a case. Alternatively, the reference may be, preferably, derived from a subject known to not suffer from a regressing prostate carcinoma, preferably from a subject suffering from progressing prostate carcinoma according to Gleason stages 3a or 3b. More preferably, the absence of the said at least one metabolite or an amount thereof which differs in the test sample in comparison to the reference sample is indicative for a regressing prostate carcinoma in these cases. It will be understood that the reference can be obtained also from a group of such subjects by measures discussed before, e.g., the median or average of the amount of the biomarker may be determined.

The present specification, furthermore, contemplates a method of determining whether a prostate carcinoma has a high or low or high, intermediate or low Gleason Score comprising
(a) determining at least one metabolite of Table 6 or 8 in a test sample of a subject suspected to suffer from prostate carcinoma of high or low or high, low or intermediate Gleason Score; and
(b) comparing the test results of the determination in step (a) to a reference, whereby it is determined whether the prostate carcinoma has a high or low or high, intermediate or low Gleason Score.

The term "determining whether a prostate carcinoma has a high or low or high, intermediate or low Gleason Score" means that a prostate carcinoma analyzed in a sample will be allocated into a category according to the Gleason Scoring system. Thus, the method allows for differentiating between Gleason Scores of different strength. The grouping of Gleason Scores into those of high and low strength for Gleason Score bisection and high, intermediate and low strength for Gleason Score trichotomy, respectively, can be found in Table 9, below. In one preferred example of the aforementioned method it is determined whether a prostate carcinoma has a high or low Gleason Score based at least one metabolite of Table 6 or 8 as indicated in the columns for Gleason Score Bi (bisection). In another preferred example of the aforementioned method it is determined whether a prostate carcinoma has a high, intermediate or low Gleason Score based at least one metabolite of Table 6 or 8 as indicated in the columns for Gleason Score Tri (trichotomy).

A reference in connection with the method described before can be obtained from a sample of a subject (or from samples of subjects) known to suffer from either a high or low Gleason Score prostate carcinoma, preferably, as indicated in Table 9 for Gleason Score Bi, below. It will be understood that if a high Gleason Score prostate carcinoma sample derived reference is applied, a test resuit that is essentially identical to the reference is indicative for a high Gleason score of the prostate carcinoma present in the test sample. A test result which differs significantly from the reference shall be indicative for a low Gleason Score of the prostate carcinoma present in the test sample. Preferred relative differences for the metabolites (i.e. up or down regulation with respect to the reference) can be derived from the information given in Table 6 or 8 for Gleason Score Bi. Moreover, preferred fold changes can also be derived from Table 6 or 8 (i.e. estimated changes for Gleason Score Bi). A test result which is essentially identical to the reference, i.e. which does not differ significantly, is indicative for a high Gleason Score of the prostate carcinoma in the sample. This applies mutatis mutandis for the high, intermediate and low Gleason Scores for Gleason Score Tri scoring according to Table 6 or 8.

Preferably, the sample in the aforementioned method is a tissue sample of the prostate carcinoma tissue or tissue suspected to comprise prostate carcinoma cells. In such a case, preferably the at least one biomarker is selected from Table 6. Also preferably, the sample is a serum sample and in such a case the at least one biomarker is, preferably, selected from Table 8.

The aforementioned-method, thus, allow for classification of the tumor, monitoring tumor progression as well as determining whether an applied therapy is successful, or not.

The present specification, furthermore, contemplates a method of determining whether a prostate carcinoma has a high or low pT Score comprising
(a) determining at least one metabolite of Table 7 in a test sample of a subject suspected to suffer from prostate carcinoma of high or low pT Score; and
(b) comparing the test results of the determination in step (a) to a reference,
whereby it is determined whether the prostate carcinoma has a high or low pT Score.

The term "determining whether a prostate carcinoma has a high or low pT Score" means that a prostate carcinoma analyzed in a sample will be allocated into a category according to the pT tumor scoring system. Thus, the method allows for differentiating between pT Scores of different strength. The grouping of pT Scores into those of high and low strength can be found in Table 9, below.

A reference in connection with the method described before can be obtained from a sample of a subject (or from samples of subjects) known to suffer from either a high or low pT Score prostate carcinoma, preferably, as indicated in Table 9 for pT Score, below. It will be understood that if a high pT Score prostate carcinoma sample derived reference is applied, a test result that is essentially identical to_the reference is indicative for a high pT score of the prostate carcinoma present in the test sample. A test result which differs significantly from the reference shall be indicative for a low pT Score of the prostate carcinoma present in the test sample. Preferred relative differences for the metabolites (i.e. up or down regulation with respect to the reference) can be derived from the information given in Table 7 for pT Score. Moreover, preferred fold changes can also be derived from Table 7 (i.e. estimated changes for pT Score). A test result which is essentially identical to the reference, i.e. which does not differ significantly, is indicative for a high pT Score of the prostate carcinoma in the sample.

Preferably, the sample in the aforementioned method is a tissue sample of the prostate carcinoma tissue or tissue suspected to comprise prostate carcinoma cells.

The aforementioned method, thus, allow for classification of the tumor, monitoring tumor progression as well as determining whether an applied therapy is successful, or not.

The method of the present invention for diagnosing prostate carcinomas or a predisposition therefor as well as the method for diagnosing a progressing or regressing prostate carcinoma can be also applied for deciding on a suitable therapy for a patient or for monitoring the success of a therapy. Accordingly, the at least one metabolite from either of the above groups in a sample of a subject can, in principle, be used for deciding on a suitable therapy for the subject or it can be used to monitor the success of such a therapy.

Therefore, the present specification further contemplates a method for determining whether a subject will benefit from a prostate carcinoma therapy comprising the steps of the method of the present invention and the further step of identifying a subject which will benefit from the prostate carcinoma therapy based on the diagnostic result, i.e. the result indicating that the subject suffers from prostate carcinoma or a predisposition therefor. Suitable prostate carcinoma therapies include surgery, low- and high-dose irradiation, hormone therapy and systemic chemotherapy, e.g., cytostatics, alone or in combination with other drugs, such as docetaxel in combination with prednisolone as frist-line.therapy, docetaxel combined with hormone therapy or with cytostatics like vinorelbine, epirubicine, capecitabine, or calcitriole. It will be understood that the method can also be applied to determine whether a subject will benefit from or is in need of a therapy against a progressing prostate carcinoma. Such a method can be applied in therapeutic approaches like "active surveillance". In this approach, a subject suffering from less advanced prostate carcinoma is subjected to a method for diagnosing progressing prostate carcinoma as set forth above on a short regular basis in order to detect the early, onset of progression. Only after the progression becomes detectable, the subject will be treated by a suitable therapy, such as surgery or radiation. Thus, "active surveillance" prevents the harmful side effects of a therapy in subjects which are - although suffering prostate carcinoma - not in an immediate need for a therapy. By avoiding the therapy at this stage, it will be understood that the harmful side effects of the therapy can be avoided as well. The "active surveillance" approach is usually practiced with younger subjects. The approach of "watchful waiting" is based on a hormonal therapy and monitoring by applying the methods on a prolonged regular basis. If no signs of progressing prostate-carcinoma are apparent, further therapeutic measures such as surgery or radiation and their side effects can be avoided (Dall'Era 2009, Curr Opin Urol 19:000-000).

The present specification, furthermore, contemplate a method for monitoring the success of a prostate carcinoma therapy or a therapy against progressing prostate carcinoma. The method shall again comprise the steps of the aforementioned method of the present invention for diagnosing prostate carcinomas or a predisposition therefor as well as the method for diagnosing a progressing prostate carcinoma and the further step of identifying a successful therapy based on the diagnostic result. It will be understood that a successful therapy shall result in a change of the at least one biomarker from a diseased or advanced into a healthy or less advanced disease stage.

As described above, in a preferred embodiment of the method of the present invention, said determining of the at least one metabolite comprises mass spectrometry (MS).

Mass spectrometry as used herein encompasses all techniques which allow for the determination of the molecular weight (i.e. the mass) or a mass variable corresponding to a compound, i.e. a metabolite, to be determined in accordance with the present invention. Preferably, mass spectrometry as used herein relates to GC-MS, LC-MS, direct infusion mass spectrometry, FT-ICR-MS, CE-MS, HPLC-MS, quadrupole mass spectrometry, any sequentially coupled mass spectrometry such as MS-MS or MS-MS-MS, ICP-MS, Py-MS. TOF or any combined approaches using the aforementioned techniques. How to apply these techniques is well known to the person skilled in the art. Moreover, suitable devices are commercially available. More preferably, mass spectrometry as used herein relates to LC-MS and/or GC-MS, i.e. to mass spectrometry being operatively linked to a prior chromatographic separation step. More preferably, mass spectrometry as used herein encompasses quadrupole MS. Most preferably, said quadrupole MS is carried out as follows: a) selection of a mass/charge quotient (m/z) of an ion created by ionisation in a first analytical quadrupole of the mass spectrometer, b) fragmentation of the ion selected in step a) by applying an acceleration voltage in an additional subsequent quadrupole which is filled with a collision gas and acts as a collision chamber, selection of a mass/charge quotient of an ion created by the fragmentation process in step b) in an additional subsequent quadrupole, whereby steps a) to c) of the method are carried out at least once and analysis of the mass/charge quotient of all the ions present in the mixture of substances as a result of the ionisation process, whereby the quadrupole is filled with collision gas but no acceleration voltage is applied during the analysis. Details on said most preferred mass spectrometry to be used in accordance with the present invention can be found in WO 03/073464.

More preferably, said mass spectrometry is liquid chromatography (LC) MS and/or gas chromatography (GC) MS.

Liquid chromatography as used herein refers to all techniques which allow for separation of compounds (i.e. metabolites) in liquid or supercritical phase. Liquid chromatography is characterized in that compounds n a mobile phase are passed through the stationary phase. When compounds pass through the stationary phase at different rates they become separated in time since each individual compound has its specific retention time (i.e. the time which is required by the compound to pass through the system). Liquid chromatography as used herein also includes HPLC. Devices for liquid chromatography are commercially available, e.g. from Agilent Technologies, USA. Gas chromatography as applied in accordance with the present invention, in principle, operates comparable to liquid chromatography. However, rather than having the compounds (i.e. metabolites) in a liquid mobile phase which is passed through the stationary phase, the compounds will be present in a gaseous volume. The compounds pass the column which may contain solid support materials as stationary phase or the walls of which may serve as or are coated with the stationary phase. Again, each compound has a specific time which is required for passing through the column. Moreover, in the case of gas chromatography it is preferably envisaged that the compounds are derivatised prior to gas chromatography. Suitable techniques for derivatisation are well known in the art. Preferably, derivatisation in accordance with the present invention relates to methoxymation and trimethylsilylation of, preferably, polar compounds and transmethylation, methoxymation and trimethylsilylation of, preferably, non-polar (i.e. lipophilic) compounds.

Furthermore, the present specification relates to a data collection comprising characteristic values of at least one metabolite being indicative for prostate carcinomas or a predisposition therefore or for a progressing prostate carcinoma, said metabolite being selected from the respective group referred to above in accordance with the methods of the present invention. The term "data collection" refers to a collection of data which may be physically and/or logically grouped together. Accordingly, the data collection may be implemented in a single data storage medium or in physically separated data storage media being operatively linked to each other. Preferably, the data collection is implemented by means of a database. Thus, a database as used herein comprises the data collection on a suitable storage medium. Moreover, the database, preferably, further comprises a database management system. The database management system is, preferably, a network-based, hierarchical or object-oriented database management system. Furthermore, the database may be a federal or - integrated database. More preferably, the database will be implemented as a distributed (federal) system, e.g. as a Client-Server-System. More preferably, the database is structured as to allow a search algorithm to compare a test data set with the data sets comprised by the data collection. Specifically, by using such an algorithm, the database can be searched for similar or identical data sets being indicative for prostate carcinoma or a predisposition thereof (e.g. a query search). Thus, if an identical or similar data set can be identified in the data collection, the test data set will be associated with prostate carcinomas or a predisposition therefor or the progression of prostate carcinoma. Consequently, the information obtained from the data collection can be used to diagnose prostate carcinomas or a predisposition therefore based on a test data set obtained from a subject. More preferably, the data collection comprises characteristic values of all metabolites comprised by any one of-the groups recited above.

In light of the foregoing, the present specification encompasses a data storage medium comprising the aforementioned data collection.

The term "data storage medium" as used herein encompasses data storage media which are based on single physical entities such as a CD, a CD-ROM, a hard disk, optical storage media, or a diskette. Moreover, the term further includes data storage media consisting of physically separated entities which are operatively linked to each other in a manner as to provide the aforementioned data collection, preferably, in a suitable way for a query search.

The present specification also relates to a system comprising:
(a) means for comparing characteristic values of metabolites of a sample operatively linked to
(b) a data storage medium as described above.

The term "system" as used herein relates to different means which are operatively linked to each other. Said means may be implemented in a single device or may be physically separated devices which are operatively linked to each other. The means for comparing characteristic values of metabolites operate, preferably, based on an algorithm for comparison as mentioned before. The data storage medium, preferably, comprises the aforementioned data collection or database, wherein each of the stored data sets being indicative for prostate carcinomas or a predisposition therefor. Thus, the system allows identifying whether a test data set is comprised by the data collection stored in the data storage medium. Consequently, the system may be applied as a diagnostic means in diagnosing prostate carcinomas or a predisposition or progression therefor.

In a preferred example of the system, means for determining characteristic values of metabolites of a sample are comprised.

The term "means for determining characteristic values of metabolites" preferably relates to the aforementioned devices for the determination of metabolites such as mass spectrometry devices, NMR devices or devices for carrying out chemical or biological assays for the metabolites.

Moreover, the present specification relates to a diagnostic means comprising means for the determination of at least one metabolite selected from any one of the groups referred to above.

The term "diagnostic means", preferably, relates to a diagnostic device, system or biological or chemical assay as specified elsewhere in the description in detail.

The expression "means for the determination of at least one metabolite," refers to devices or agents which are capable of specifically recognizing the metabolite. Suitable devices may be spectrometric devices such as mass spectrometry, NMR devices or devices for carrying out chemical or biological assays for the metabolites. Suitable agents may be compounds which specifically detect the metabolites. Detection as used herein may be a two-step process, i.e. the compound may first bind specifically to the metabolite to be detected and subsequently generate a detectable signal, e.g., fluorescent signals, chemiluminescent signals, radioactive signals and the like. For the generation of the detectable signal-further compounds may be required which are all comprised by the term "means for determination of the at least one metabolite". Compounds which specifically bind to the metabolite are described elsewhere in the specification in detail and include, preferably, enzymes, antibodies, ligands, receptors or other biological molecules or chemicals which specifically bind to the metabolites. In a preferred example the detectable signal also represent a quantifiable signal, meaning the relative intensity of the at least one metabolite is proportional to the relative intensity of the detectable signal.

Further, the present specification relates to a diagnostic composition comprising at least one metabolite selected from any one of the groups referred to above.

The at least one metabolite selected from any of the aforementioned groups will serve as a biomarker, i.e. an indicator molecule for a pathological condition or predisposition in the subject, i.e. prostate carcinomas or a predisposition therefor. Thus, the metabolites itself may serve as diagnostic compositions, preferably, upon visualization or detection by the means referred to in herein. Thus, a diagnostic composition which indicates the presence of a metabolite may also comprise the said biomarker physically, e.g., a complex of an antibody and the metabolite to be detected may serve as the diagnostic composition. Accordingly, the diagnostic composition may further comprise means for detection of the metabolites as specified elsewhere in this description. Alternatively, if detection means such as MS or NMR based techniques are used, the molecular species which serves as an indicator for the pathological condition will be the at least one metabolite comprised by the test sample to be investigated. Thus, the at least one metabolite referred to in accordance with the present invention shall serve itself as a diagnostic composition due to its identification as a biomarker.

Finally, the present invention relates to the use of at least one metabolite or a compound which specifically detects said metabolite for diagnosing prostate carcinomas, a predisposition therefore or a progressing prostate carcinoma in a sample of a subject, said metabolite being selected from the respective group referred to above in accordance with the methods of the present invention.

As specified above already, each of said metabolites is a suitable biomarker by its own for the diseases referred to herein. However, most preferably, a group of biomarkers including biomarkers of any one of the aforementioned groups is to be determined by the method of the present invention. A group of biomarkers consists, preferably, of at least two at least three, at least four and, preferably, up to all of the aforementioned biomarkers.

The invention will now be illustrated by the following Examples which are not intended to restrict or limit the scope of this invention.

### Example 1: Determination of metabolites

Biomarkers were discovered by analyzing tissue and partly serum samples from the same group of human subjects to determine the levels of metabolites in the samples and then statistically analyzing the results to determine those metabolites that are the same or different in tissue or serum.

The cancer and control tissue from 107 subjects suffering from prostate cancer as well as serum samples from a subset of 64 subjects were used for the analysis. Additional clinical information for all subjects (e.g. age, BMI, medication, date of sampling, DRUS, TRUS, total PSA, free PSA, ratio f/t PSA, tumor-status pT, Gleason [at all] Gleason [punch] were included in the analysis

Samples were prepared and subjected to LC-MS/MS and GC-MS or for human serum samples XLC-MS/MS (hormones) analysis as described in the following:

The sample were prepared in the following way: Proteins were separated by precipitation from blood serum or from extracts obtained solved extraction of the freeze-dried tissue material . After addition of water and a mixture of ethanol and dichlormethan the remaining sample was fractioned into an aqueous, polar phase (Polar fraction) and an organic, lipophilic phase (lipid fraction).

For the transmethanolysis of the lipid extracts a mixture of 140 µl of chloroform, 37 µl of -hydrochloric-acid (37% by weight HCl in water), 320 µl of methanol and 20 µl of toluene was added to the evaporated extract. The vessel was sealed tightly and heated for 2 hours at 100°C, with shaking. The solution was subsequently evaporated to dryness. The residue was dried completely.

The methoximation of the carbonyl groups was carried out by reaction with methoxyamine hydrochloride (20 mg/ml in pyridine, 100 µl for 1.5 hours at 60°C) in a tightly sealed vessel. 20 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 100 µl of N-methyl-N-(trimethylsilyl)-2,2,2-trifluoroacetamide (MSTFA) was carried out for 30 minutes at 60°C, again in the tightly sealed vessel. The final volume before injection into the GC was 220 µl.

For the polar phase the derivatization was performed in the following way: The methoximation of the carbonyl groups was carried out by reaction with methoxyamine hydrochloride (20 mg/ml in pyridine, 50 µl for 1.5 hours at 60°C) in a tightly sealed vessel. 10 µl of a solution of odd-numbered, straight-chain fatty acids (solution of each 0.3 mg/mL of fatty acids from 7 to 25 carbon atoms and each 0.6 mg/mL of fatty acids with 27, 29 and 31 carbon atoms in 3/7 (v/v) pyridine/toluene) were added as time standards. Finally, the derivatization with 50 µl of N-methyl-N-(trimethylsilyl)-2,2,2-trifluoroacetamide (MSTFA) was carried out for 30 minutes at 60°C, again in the tightly sealed vessel. The final volume before injection into the GC was 110 µl.

The GC-MS systems consist of an Agilent 6890 GC coupled to an Agilent 5973 MSD. The autosamplers are CompiPal or GCPal from CTC.

For the analysis usual commercial capillary separation columns (30 m x 0,25 mm x 0,25 µm) with different poly-methyl-siloxane stationary phases containing 0 % up to 35% of aromatic moieties, depending on the analysed sample materials and fractions from the phase separation step, were used (for example: DB-1ms, HP-5ms, DB-XLB, DB-35ms, Agilent Technologies). Up to 1 µL of the final volume was injected splitless and the oven temperature program was started at 70 °C and ended at 340 °C with different heating rates depending on the sample material and fraction from the phase separation step in order to achieve a sufficient chromatographic separation and number of scans within each analyte peak. Furthermore RTL (Retention Time Locking, Agilent Technologies) was used for the analysis and usual GC-MS standard conditions, for example constant flow with nominal 1 to 1.7 ml/min. and helium as the mobile phase gas, ionisation was done by electron impact with 70 eV, scanning within a m/z range from 15 to 600 with scan rates from 2.5 to 3 scans/sec and standard tune conditions.

The HPLC-MS systems consisted of an Agilent 1100 LC system (Agilent Technologies, Waldbronn, Germany) coupled with an API 4000 Mass spectrometer (Applied Biosystem/MDS SCIEX, Tororito, Canada). HPLC analysis was performed on commercially available reversed phase separation columns with C18 stationary phases (for example: GROM ODS 7 pH, Thermo Betasil C18). Up to 10 µL of the final sample volume of evaporated and reconstituted polar and lipophilic phase was injected and separation was performed with gradient elution using methanol/water/formic acid or acetonitrile/water/formic acid gradients at a flowrate of 200 µL/min.

Mass spectrometry was carried out by electrospray ionisation in positive mode for the nonpolar fraction (lipid fraction) and negative mode for the polar fraction using multiple-reaction-monitoring-(MRM)-mode and fullscan from 100- 1000 amu.

Steroids and their metabolites were measured by online SPE-LC-MS (Solid phase extraction-LC-MS). Catecholamines and their metabolites were measured by online SPE-LC-MS as described by Yamada et al [21].

### Example 2: Data evaluation

Serum samples were analyzed in randomized analytical sequence design with pooled samples (so called "Pool") generated from aliquots of each sample. The raw peak data were normalized to the median of pool per analytical sequence to account for process variability (so called "ratios"). Ratios were log10 transformed to approach a normal distribution of data. Statistical analysis was done by a linear model correcting data for BMI, age and storage time and predicting total PSA, free PSA, ratio f/t PSA, tumor-status pT, Gleason [at all] Gleason [punch]. Metabolites from this analysis were ideln addition, a Receiver Operating Characteristic (ROC) analysis was performed to calculate Area Under Curve (AUC) for progression of tumor status from phase 2 to phase 3.

Prostate tissue samples were analyzed in semi-randomized analytical sequence design (samples of each subject analyzed in subsequent slots, subjects and sequence of tissue randomized) with pooled samples (= "pool") generated from extra samples provided for this purpose. The raw peak data were normalized to the median of pool per analytical sequence to account for process variability (so called "ratios versus pool"). Ratios versus pool were re-centered with two different methods: 1) Normalization to the median of all control samples to focus on cancer-induced changes by retaining variability of control group. 2) Normalization of cancer sample ratios intra-individually to corresponding control sample to account for inter-individual variability.

All ratios were log10 transformed to approach a normal distribution of data.

Statistical analysis was performed on log10 transformed ratios from normalization method 1 by a paired two-sided t-test and on log10 transformed ratios from normalization method 2 by linear regression analysis with total PSA, free PSA, ratio f/t PSA, tumor-status pT, Gleason [at all] Gleason [punch]. In addition, a Receiver Operating Characteristic (ROC) analysis was performed to calculate Area Under Curve (AUC) for cancer tissue versus healthy control on ratios from normalization method 1.

Data integration of serum and prostate tissue samples was done with serum ratios and prostate tissue ratios from intra-individual normalization of cancer sample to corresponding control. Statistical analysis was done by linear regression of all metabolites from serum with all metabolites from prostate tissue. From the correlation matrix obtained, biomarker candidates were identified with 3 methods:
1) Metabolite in serum data showing a significant correlation with the same metabolite in prostate tissue data and therefore a biomarker candidate in serum for prostate cancer diagnosis
2) Metabolite in serum showing a significant correlation (i.e. being among the top 40 most significant correlations based on p-value of linear regression) with prostate tissue data and therefore a biomarker candidate in serum for prostate cancer diagnosis.
3) Metabolite in serum showing a significant correlation with one of the 7 most significantly changed prostate tissue metabolites (7-methylguanine, biotin, glycine, hypoxanthine, tricosanoic acid, MetID(69800140), uridine) and therefore a biomarker candidate in serum for prostate cancer diagnosis.

The results of the data evaluation are shown in the following tables.

**Tab. 1: Metabolite biomarkers in prostate cancer tissue with a significantly different concentration level compared to healthy tissue based on a pair-wise two-sided t-test..**

| Metabolite | Kind of regulation | Median of carcinom tissue relative to control | p-value of paired t-test, two-sided | AUC carcinom versus healthy tissue |
|---|---|---|---|---|
| 7-Methylguanine | up | 1,55 | 0,0000 | 0,75 |
| 2-Hydroxybehenic acid (C22:0) | up | 6,62 | 0,0000 | 0,85 |
| Cerebronic acid (2-OH-C24:0) | up | 3,73 | 0,0000 | 0,82 |
| Isopentenyl pyrophosphate (IPP) | up | 1,45 | 0,0000 | 0,75 |
| 14-Methylhexadecanoic acid | up | 1,14 | 0,0000 | 0,62 |
| 2-Aminoadipinic acid | up | 1,45 | 0,0000 | 0,68 |
| Ceramide (d18:1, C24:1) | up | 1,10 | 0,0011 | 0,60 |
| Eicosenoic acid (C20:cis[11]1) | up | 1,55 | 0,0000 | 0,70 |
| Tricosanoic acid (C23:0) | up | 2,99 | 0,0000 | 0,85 |
| Glycerophosphoethanolamine, polar fraction | up | 2,50 | 0,0000 | 0,75 |
| Eicosadienoic acid (C20:2) No 02 | up | 1,53 | 0,0000 | 0,72 |
| Arginine | up | 1,18 | 0,0000 | 0,62 |
| Behenic acid (C22:0) | up | 1,37 | 0,0000 | 0,69 |
| beta-Carotene | up | 1,25 | 0,0000 | 0,60 |
| Cholestenol No 02 | up | 1,33 | 0,0000 | 0,69 |
| Cytosine | up | 1,14 | 0,0000 | 0,66 |
| DAG (C18:1, C18:2) | up | 1,04 | 0,0020 | 0,56 |
| Dihydrocholesterol | up | 1,35 | 0,0000 | 0,66 |
| Erythro-Dihydrosphingosine | up | 1,12 | 0,0056 | 0,58 |
| Docosahexaenoic acid (C22:cis[4,7,10,13,16,19]6) | up | 1,24 | 0,0000 | 0,65 |
| Dodecanol | up | 1,08 | 0,0323 | 0,57 |
| Eicosanoic acid (C20:0) | up | 1,41 | 0,0000 | 0,68 |
| Eicosapentaenoic acid (C20:cis[5,8,11,14,17]5) | up | 1,20 | 0,0003 | 0,60 |
| Dihomo-gamma-Linolenic acid (C20:cis[8,11,14]3) | up | 1,20 | 0,0000 | 0,64 |
| erythro-C16-Sphingosine | up | 1,45 | 0,0000 | 0,69 |
| Flavine adenine dinucleotide (FAD) | up | 1,35 | 0,0000 | 0,69 |
| gamma-Tocopherol | up | 1,37 | 0,0000 | 0,67 |
| Gluconic acid | down | 0,59 | 0,0002 | 0,62 |
| Glucuronic acid | down | 0,67 | 0,0001 | 0,62 |
| Glycerol-2-phosphate | up | 1,48 | 0,0000 | 0,74 |
| Lignoceric acid (C24:0) | up | 1,33 | 0,0000 | 0,68 |
| Lysophosphatidylcholine (C18:2) | up | 1,16 | 0,0000 | 0,62 |
| Lysophosphatidylcholine (C20:4) | up | 1,06 | 0,0007 | 0,57 |
| Maltotriose | down | 0,54 | 0,0000 | 0,62 |
| myo-Inositol, lipid fraction | up | 1,13 | 0,0007 | 0,63 |
| myo-Inositol-2-phosphate, lipid fraction (myo-Inositolphospholipids) | up | 1,44 | 0,0000 | 0,70 |
| Nervonic acid (C24:cis[15]1) | up | 1,15 | 0,0002 | 0,62 |
| Nicotinamide | up | 1,13 | 0,0004 | 0,61 |
| Pentadecanol | up | 1,52 | 0,0000 | 0,75 |
| Phosphatidylcholine (C18:0, C22:6) | down | 0,88 | 0,0004 | 0,62 |
| Phytosphingosine | up | 1,24 | 0,0002 | 0,64 |
| Pseudouridine | up | 1,10 | 0,0005 | 0,62 |
| Pyruvate | up | 1,07 | 0,0004 | 0,60 |
| 3-O-Methylsphingosine | up | 1,33 | 0,0001 | 0,67 |
| threo-Sphingosine | up | 1,33 | 0,0001 | 0,68 |
| 5-O-Methylsphingosine | up | 1,30 | 0,0002 | 0,66 |
| erythro-Sphingosine | up | 1,32 | 0,0012 | 0,64 |
| Sphingosine-1-phosphate | up | 1,26 | 0,0005 | 0,65 |
| Threonic acid | up | 1,17 | 0,0011 | 0,60 |
| Sphingosine Isomer No 01 | up | 1,38 | 0,0001 | 0,66 |

**Tab. 2: Metabolites in serum showing a significantly different concentration level when cancer progresses from phase 2 to phase 3 and providing biomarkers in serum for prostate cancer progression diagnosis.**

| | Kind of regulaion | Tumor status 3a,3b versus 2 | Tumor status 3a,3b versus 2 | Tumor status 3a,3b versus 2 | p-Value |
|---|---|---|---|---|---|
| Metabolite | | t-value | p-value | AUC | |
| MetID(58300131) | down | -2,427 | 0,0186 | 0,57 | <0.05 |
| Phosphatidylcholine (C18:0,C18:2) | up | 2,157 | 0,0356 | 0,64 | <0.05 |
| Phosphatidylcholine (C16:0, C20:4) | down | -2,280 | 0,0268 | 0,63 | <0.05 |
| 2-Oxoisocaproic acid | down | -1,175 | 0,2454 | 0,52 | <0.25 |
| Erythronic acid | down | -1,445 | 0,1544 | 0,55 | <0.25 |
| Choline plasmalogen (C18,C20:4) | down | -1,191 | 0,2389 | 0,56 | <0.25 |
| MetID(68300015) | down | -1,177 | 0,2445 | 0,55 | <0.25 |
| MetID(68300047) | down | -2,054 | 0,0450 | 0,62 | <0.05 |
| Lysophosphatidylcholine (18:0) | down | -1,226 | 0,2257 | 0,59 | <0.25 |
| Phosphatidylcholine (C16:0, C16:0) | up | 1,169 | 0,2476 | 0,60 | <0.25 |
| Phosphatidylcholine (C18:0, C20:4) | down | -1,544 | 0,1287 | 0,53 | <0.15 |
| MetID(68300020) | down | -1,262 | 0,2124 | 0,58 | <0.25 |
| Pyruvate | down | -1,204 | 0,2339 | 0,54 | <0.25 |
| beta-Sitosterol | down | -1,626 | 0,1100 | 0,62 | <0.15 |
| Coenzyme Q10 | down | -1,548 | 0,1277 | 0,59 | <0.15 |

**Tab. 3 Metabolites identified in Serum in comparison to prostata tissue**

| Metabolite | t-value | p-value | Data Analysis approach |
|---|---|---|---|
| Choline plasmalogen (C18, C20:4) | 3,007 | 0,0042 | fPSA_tPSA_log Ratio Effect |
| 2-Oxoisocaproic acid | increase | 0,0253 | Correlation analysis tissue and serum, top 7 prostate cancer biomarker |
| | | | |
| MetID(68300015) | decrease | 0,00014 | Correlation analysis tissue and serum, TOP 40 correlation |
| MetID(68300047) | -2,390 | 0,0205 | Tumor status numerical |
| Erythronic acid | increase | 0,0070 | Correlation analysis tissue and serum, top 7 prostate cancer biomarker |
| Behenic acid (C22:0) | decrease | 0,00052 | Correlation analysis tissue and serum, TOP 40 correlation |
| | | | |
| myo-Inositol-2-phosphate, lipid fraction (myoInositolphospilolipids) | decrease | 0,00054 | Correlation analysis tissue and serum, TOP 40 correlation |
| MetID(38300600) | 3,314 | 0,0018 | Iog tPSA Effect |
| 1,5-Anhydrosorbitol | -2,072 | 0,0432 | Tumor status numerical |
| 14-Methylhexadecanoic acid | 2,427 | 0,0188 | Gleasongesamt_sum |
| 3-Hydroxybutyrate | 2,853 | 0,0064 | log fPSA Effect |
| 3-Methoxytyrosine | -1,728 | 0,0897 | Gleason_Stanze_Sum |
| | | | |
| 4-Hydroxy-3-methoxyphenylglycol (HMPG) | decrease | 0,00059 | Correlation analysis tissue and serum, TOP 40 correlation |
| | | | |
| 5-Hydroxy-3-indoleacetic acid (5-H IAA) | 1,723 | 0,0907 | log tPSA Effect |
| beta-Carotene | increase | 0,0325 | Correlation analysis tissue and serum, top 7 prostate cancer biomarker |
| beta-Sitosterol | increase | 0,00044 | Correlation analysis tissue and serum, TOP 40 correlation |
| Canthaxanthin | 2,032 | 0,0479 | log fPSA Effect |
| Ceramide (d18:1, C24:0) | increase | 0,0468 | Correlation analysis tissue and serum, top 7 prostate cancer biomarker |
| Cholestenol No 02 | increase | 0,0309 | Correlation analysis tissue and serum, top 7 prostate cancer biomarker |
| MetID(68300017) | 1,965 | 0,0548 | Gleasongesamt_sum |
| Coenzyme Q10 | -2,013 | 0,0493 | Tumor status numerical |
| conjugated Linoleic acid (C18:trans[9,11]2) | 2,093 | 0,0414 | Gleasongesamt_sum |
| Cryptoxanthin | increase | 0,00048 | Correlation analysis tissue and serum, TOP 40 correlation |
| | | | |
| Dihydrotestosterone | 1,894 | 0,0642 | fPSA_tPSA_log Ratio Effect |
| | | | |
| Docosahexaenoic acid (C22:cis[4,7,10,13,16,19]6) | increase | 0,0300 | Correlation analysis tissue and serum, top 7 prostate cancer biomarker |
| Dodecanol | -1,934 | 0,0592 | fPSA_tPSA_log Ratio Effect |
| Eicosanoic acid (C20:0) | increase | 0,0290 | Correlation analysis tissue and serum, top 7 prostate cancer biomarker |
| Eicosapentaenoic acid (C20:cis[5,8,11,14,17]5) | increase | 0,0041 | Correlation analysis tissue and serum, top 7 prostate cancer biomarker |
| Dihomo-gamma-Linolenic acid (C20:cis[8,11,14]3) | increase | 0,00022 | Correlation analysis tissue and serum, TOP 40 correlation |
| erythro-C16-Sphingosine | increase | 0,0363 | Correlation analysis tissue and serum, top 7 prostate cancer biomarker |
| gamma-Linolenic acid (C 18: cis[6,9,12]3) | increase | 0,0176 | Correlation analysis tissue and serum, top 7 prostate cancer biomarker |
| Glycerate | -2,216 | 0,0316 | fPSA_tPSA_log Ratio Effect |
| Lactate | -2,644 | 0,0111 | fPSA_tPSA_log Ratio Effect |
| Lignoceric acid (C24:0) | increase | 0,0423 | Correlation analysis tissue and serum, top 7 prostate cancer biomarker |
| | | | |
| Lycopene | increase | 0,0412 | Correlation analysis tissue and serum, top 7 prostate cancer biomarker |
| Lysophosphatidylcholine (16:0) | 2,148 | 0,0365 | log tPSA Effect |
| Lysophosphatidylcholine (C18:2) | decrease | 0,00048 | Correlation analysis tissue and serum, TOP 40 correlation |
| Lysophosphatidylcholine (C17:0) | 1,916 | 0,0610 | log tPSA Effect |
| Nervonic acid (C24:cis[15]1) | increase | 0,0410 | Correlation analysis tissue and serum, top 7 prostate cancer biomarker |
| Phosphatidylcholine (C16:0, C20:4) | | 0,0409 | Tumor status numerical |
| Phosphatidylcholine (C18:0, C18:2) | 1,707 | 0,0938 | Gleason_Stanze_Sum |
| Phosphatidylcholine (C18:2, C20:4) | -1,756 | 0,0856 | fPSA_tPSA_log Ratio Effect |
| | | | |
| MetID(68300048) | increase | 0,00044 | Correlation analysis tissue and serum, TOP 40 correlation |
| | | | |
| Phosphatidylcholine No 02 | increase | 0,0087 | Correlation analysis tissue and serum, top 7 prostate cancer biomarker |
| Phosphatidylcholine (C18:0, C20:4) | -2,375 | 0,0213 | Tumor status numerical |
| | | | |
| MetID(68300020) | 2,250 | 0,0292 | log fPSA Effect |
| Pyruvate | -2,193 | 0,0327 | Tumor status numerical |
| scyllo-Inositol | -2,945 | 0,0050 | fPSA_tPSA_log Ratio Effect |
| 3-O-Methylsphingosine ( | decrease | 0,00000 | Correlation analysis tissue and serum, TOP 40 correlation |
| 5-O-Methylsphingosine | decrease | 0,00001 | Correlation analysis tissue and serum, TOP 40 correlation |
| erythro-Sphingosine | decrease | 0,00001 | Correlation analysis tissue and serum, TOP 40 correlation |
| | | | |
| Sphingomyelin (d18:1, C16:0) | decrease | 0,00031 | Correlation analysis tissue and serum, TOP 40 correlation |
| MetID(68300045) | 2,281 | 0,0268 | log tPSA Effect |
| Testosterone | 1,694 | 0,0962 | log tPSA Effect |
| Dehydroepiandrosterone sulfate | 2,310 | 0,0248 | Gleasongesamt sum |
| Threonic acid | -2,468 | 0,0173 | fPSA_tPSA_log Ratio Effect |
| Tricosanoic acid (C23:0) | increase | 0,0389 | Correlation analysis tissue and serum, top 7 prostate cancer biomarker |

**Table 4: Detailed explanation of the different data analysis approaches mentioned in table 3.**

| **Data Analysis approach** | **Explanation** |
|---|---|
| Correlation analysis tissue and serum, significant correlation of same metabolite | Metabolite in serum data showing a significant correlation with the same metabolite in prostate tissue data and therefore a biomarker candidate in serum for prostate cancer diagnosis. Metabolite data of prostate cancer tissue analysis was intra-individually normalized to prostate healthy tissue data and serum data was normalized to pooled samples. |
| Correlation analysis tissue and serum, TOP 40 correlation | Metabolite in serum showing a significant correlation (i.e. being among the top 40 most significant correlations based on p-value of linear regression) with prostate tissue data and therefore a biomarker candidate in serum for prostate cancer diagnosis. Metabolite data of prostate cancer tissue analysis was intra-individually normalized to prostate healthy tissue data and serum data was normalized to pooled samples. |
| Correlation analysis tissue and serum, top 7 prostate cancer biomarker | Metabolite in serum showing a significant correlation with one of the 7 most significantly changed prostate tissue metabolites (7-methylguanine, biotin, glycine, hypoxanthine, tricosanoic acid, MetID(69800140), uridine) and therefore a biomarker candidate in serum for prostate cancer diagnosis. Metabolite data of prostate cancer tissue analysis was intra-individually normalized to prostate healthy tissue data and serum data was normalized to pooled samples. |
| fPSA_tPSA_log Ratio Effect | Metabolite biomarker candidate in serum for prostate cancer diagnosis. Result of a linear model correlating the log10 transformed ratio of fPSA to tPSA (ratio of free prostate specific antigen data to total prostate specific antigen data) with the log10 tansformed serum metabolite data (ratios versus pooled samples), corrected for age, BMI and storage duration effects |
| Gleason_Stanze_Sum | Metabolite biomarker candidate in serum for prostate cancer diagnosis. Result of a linear model correlating the Gleason score of the prostate cancer biopsy with the log10 tansformed serum metabolite data (ratios versus pooled samples), corrected for age, BMI and storage duration effects |
| Gleasongesamt_sum | Metabolite biomarker candidate in serum for prostate cancer diagnosis. Result of a linear model correlating the Gleason score of the entire prostate cancer after surgery with the log10 tansformed serum metabolite data (ratios versus pooled samples), corrected for age, BMI and storage duration effects |
| log fPSA Effect | Metabolite biomarker candidate in serum for prostate cancer diagnosis. Result of a linear model correlating the log10 transformed fPSA (free prostate specific antigen) data with the log10 tansformed serum metabolite data (ratios versus pooled samples), corrected for age, BMI and storage duration effects |
| log tPSA Effect | Metabolite biomarker candidate in serum for prostate cancer diagnosis. Result of a linear model correlating the log10 transformed tPSA (total prostate specific antigen) data with the log10 tansformed serum metabolite data (ratios versus pooled samples), corrected for age, BMI and storage duration effects |
| Tumor status numerical | Metabolite biomarker candidate in serum for prostate cancer diagnosis. Result of a linear model correlating the tumor status score of the entire prostate cancer after surgery with the log10 tansformed serum metabolite data (ratios versus pooled samples), corrected for age, BMI and storage duration effects |

**Table 5 : Chemical/physical properties of MetIDs and selected metabolites. The biomarkers defined by a MetID number in the previous tables are characterized by chemical and physical properties.**

| **Met ID** | Characteriscs |
|---|---|
| 68300012 | Metabolite 68300012 is present in human serum and if detected with LC/MS, applying electro-spray ionization (ESI) mass spectrometry, the mass-to-charge ratio (m/z) of the positively charged ionic species is 808.4 (+/- 0.5). |
| 68300015 | Metabolite 68300015 belongs to the class of choline plasmalogens. It exhibits the following characteristic ionic species when detected with LC/MS, applying electro-spray ionization (ESI) mass spectrometry: mass-to-charge ratio (m/z) of the positively charged ionic species is 767 (+/- 0.5). |
| 68300017 | Metabolite 68300017 belongs to the class of choline plasmalogens. It exhibits the following characteristic ionic species when detected with LC/MS, applying electro-spray ionization (ESI) mass spectrometry: mass-tocharge ratio (m/z) of the positively charged ionic species is 772.6 (+/- 0.5). |
| 68300020 | Metabolite 68300020 belongs to the class of glycerophosphatidylcholines. It exhibits the following characteristic ionic species when detected with LC/MS, applying electro-spray ionization (ESI) mass spectrometry: mass-to-charge ratio (m/z) of the positively charged ionic species is 796.8 (+/- 0.5). |
| 68300045 | Metabolite 68300045 belongs to the class of diacylglycerides. It exhibits the following characteristic ionic species when detected with LC/MS, applying electro-spray ionization (ESI) mass spectrometry: mass-to-charge ratio (m/z) of the positively charged ionic species is 695.6 (+/- 0.5). |
| 68300047 | Metabolite 68300047 belongs to the class of choline plasmalogens. It exhibits the following characteristic ionic species when detected with LC/MS, applying electro-spray ionization (ESI) mass spectrometry: mass-to-charge ratio (m/z) of the positively charged ionic species is 768.6 (+/- 0.5). |
| 68300048 | Metabolite 68300048 belongs to the class of glycerophosphatidylcholines. It exhibits the following characteristic ionic species when detected with LC/MS, applying electro-spray ionization (ESI) mass spectrometry: mass-to-charge ratio (m/z) of the positively charged ionic species is 780.8 (+/- 0.5). |
| 58300131 | Metabolite 58300131 exhibits the following characteristic ionic species when detected with LC/MS, applying electro-spray ionization (ESI) mass spectrometry: mass-to-charge ratio (m/z) of the negatively charged ionic species is 127 (+/- 0.5). |
| 38300600 | Metabolite 38300600 exhibits the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 73 (100), 375 (38), 147 (27), 217 (19), 257 (14), 376 (14), 169 (14), 75 (10), 250 (7), 133 (7). |
| 5-O-Methylsphingosine | 5-O-Methylsphingosine exhibits the following characteristic ionic fragments when detected with GC/MS, applying electron impact (EI) ionization mass-spectrometry, after acidic methanolysis and derivatisation with 2% Omethylhydroxylamine-hydrochlorid in pyridine and subsequently with N-methyl-N-trimethylsilyltrifluoracetamid: MS (EI, 70 eV): m/z (%): 250 (100), 73 (34), 251 (19), 354 (14), 355 (4), 442 (1). |
| Cholestenol No 02 | Cholestenol No 02 represents a Cholestenol isomer. It exhibits the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 143 (100), 458 (91), 73 (68), 81 (62), 95 (36), 185 (23), 327 (23), 368 (20), 255 (15), 429 (15). |
| 3-O-Methylsphingosine | 3-O-Methylsphingosine exhibits the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 204 (100), 73 (18), 205 (16), 206 (7), 354 (4), 442 (1). |
| Eicosadienoic acid (C20:2) No 02 | Eicosadienoic acid (C20:2) No 02 represents an Eicosadienoic acid isomer. It exhibits the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 81 (100), 57 (98), 43 (92), 67 (85), 41 (80), 55 (74), 82 (66), 95 (64), 110 (39), 109 (39). |
| Phosphatidylcholine No 02 | Phosphatidylcholine No 02 belongs to the class of glycerophosphatidylcholines. It exhibits the following characteristic ionic species when detected with LC/MS, applying electro-spray ionization (ESI) mass spectrometry: mass-to-charge ratio (m/z) of the positively charged ionic species is 808.4 (+/- 0.5). |
| Sphingosine isomer No 01 | Sphingosine isomer No 01 represents a Sphingosine isomer. It exhibits the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 73 (100), 250 (37), 412 (37), 147 (21), 322 (14), 413 (11), 128 (9), 251 (7), 500 (2). |

A further approach of data analysis aimed at the metabolic differentiation of subjects with high Gleason Score prostate cancer and low Gleason Score prostate cancer (Bisection of Gleason Score, abbreviated as Gleason Bi in Table 6 and Table 8). A similar approach was performed with a Gleason trichotomy (abbreviated as Gleason Tri in Table 6 and Table 8). Both analysis were applied on both prostate tissue data and serum data. A similar approach was performed with the tumor status pT (Table 7) on prostate tissue data. Details of classification of subjects into Gleason Bi, Gleason Tri and pT score low/high are given in Table 9. Differentiating metabolites were identified with linear models (ANOVA) and given in Tables 6, 7, 8). Estimated changes >1 indicate up-regulation, estimated changes <1 indicate down-regulation.

**Table 6: Metabolites that differentiate between different levels of Gleason score in prostate cancer tissue. Estimated changes and p-values were calculated by a mixed-effect linear model (ANOVA with subject as random factor) on log10 transformed ratios. Criteria of classification are given in Table 9.**

| | Gleason Score Bi (high versus low) | Gleason Score Tri (high versus intermediate) | Gleason Score Tri (high versus low) | Gleason Score Tri (intermediate versus low) |
|---|---|---|---|---|
| Metabolite | Estimated change | Estimated change | Estimated change | Estimated change |
| 2-Hydroxybehenic acid (C22:0) | 1.474 | 1.040 | 1.509 | 1.450 |
| Cerebronic acid (2-OH-C24:0) | 1.471 | 1.201 | 1.640 | 1.365 |
| Pentadecanol | 1.266 | 1.264 | 1.453 | 1.150 |
| Pseudouridine | 1.196 | 1.082 | 1.256 | 1.161 |
| myo-Inositol, lipid fraction | 1.195 | 1.283 | 1.386 | 1.080 |
| 14-Methylhexadecanoic acid | 1.173 | 1.514 | 1.501 | 0.992 |
| Eicosapentaenoic acid (C20:cis[5,8,11,14,17]5) | 1.165 | 1.542 | 1.506 | 0.976 |
| Arginine | 1.153 | 1.292 | 1.337 | 1.035 |
| Erythronic acid | 1.142 | 0.996 | 1.139 | 1.144 |
| Tricosanoic acid (C23:0) | 1.139 | 1.201 | 1.270 | 1.057 |
| myo-Inositol-2-phosphate, lipid fraction | 1.129 | 1.352 | 1.360 | 1.006 |
| 7-Methylguanine | 1.120 | 1.262 | 1.286 | 1.019 |
| Isopentenyl pyrophosphate (IPP) | 1.114 | 1.247 | 1.269 | 1.017 |
| erythro-C16-Sphingosine | 1.114 | 1.139 | 1.204 | 1.057 |
| Glycerophosphoethanolamin e, polar fraction | 1.102 | 1.677 | 1.505 | 0.898 |
| Dihydrocholesterol | 1.091 | 2.044 | 1.661 | 0.813 |
| Cholestenol No 02 | 1.090 | 1.399 | 1.331 | 0.951 |
| threo-Sphingosine | 1.088 | 1.112 | 1.157 | 1.041 |
| Glycerol-2-phosphate | 1.081 | 1.547 | 1.411 | 0.912 |
| Pyruvate | 1.063 | 1.086 | 1.119 | 1.030 |
| Threonic acid | 1.053 | 1.302 | 1.237 | 0.951 |
| Docosahexaenoic acid (C22:cis[4, 7,10,13,16,19]6) | 1.053 | 1.352 | 1.256 | 0.929 |
| Sphingosine isomer No 01 | 1.048 | 1.190 | 1.166 | 0.980 |
| 3-O-Methylsphingosine | 1.046 | 1.124 | 1.122 | 0.998 |
| erythro-Dihydrosphingosine | 1.041 | 0.992 | 1.036 | 1.044 |
| 5-O-Methylsphingosine | 1.037 | 1.127 | 1.114 | 0.989 |
| myo-Inositol-2-phosphate | 1.033 | 0.968 | 1.012 | 1.046 |
| erythro-Sphingosine | 1.019 | 1.114 | 1.087 | 0.976 |
| Nicotinamide | 1.018 | 1.152 | 1.110 | 0.963 |
| Cytosine | 1.013 | 1.006 | 1.016 | 1.010 |
| Flavine adenine dinucleotide (FAD) | 1.012 | 1.217 | 1.154 | 0.948 |
| Lignoceric acid (C24:0) | 1.011 | 1.281 | 1.170 | 0.913 |
| Nervonic acid (C24:cis[15]1) | 1.007 | 1.151 | 1.097 | 0.953 |
| DAG (C18:1,C18:2) | 1.006 | 1.036 | 1.028 | 0.992 |
| Sphingosine-1-phosphate | 1.002 | 1.153 | 1.090 | 0.946 |
| Behenic acid (C22:0) | 0.998 | 1.181 | 1.102 | 0.933 |
| Nicotinic acid | 0.995 | 1.073 | 1.036 | 0.965 |
| Phytosphingosine, total | 0.992 | 1.153 | 1.079 | 0.936 |
| Eicosanoic acid (C20:0) | 0.990 | 1.209 | 1.108 | 0.917 |
| dihomo-gamma-Linolenic acid (C20:cis[8,11,14]3) | 0.984 | 1.308 | 1.156 | 0.884 |
| Sedoheptulose-7-phosphate | 0.980 | 1.015 | 0.989 | 0.974 |
| Phosphocreatine | 0.969 | 1.235 | 1.099 | 0.890 |
| Glycolate | 0.965 | 0.912 | 0.913 | 1.002 |
| beta-Sitosterol | 0.964 | 0.927 | 0.923 | 0.995 |
| Sphingomyelin (d18:1,C24:0) | 0.964 | 0.937 | 0.927 | 0.990 |
| Sphingomyelin (d18:1,C16:0) | 0.963 | 0.934 | 0.925 | 0.990 |
| Phosphatidylcholine (C18:0,C18:1) | 0.953 | 0.936 | 0.917 | 0.980 |
| Ceramide (d18:1,C24:1) | 0.953 | 1.071 | 0.992 | 0.926 |
| Lysophosphatidylcholine (C20:4) | 0.952 | 1.109 | 1.012 | 0.913 |
| Phosphatidylcholine (C16:0,C20:4) | 0.950 | 0.940 | 0.916 | 0.975 |
| gamma-Tocopherol | 0.950 | 1.267 | 1.097 | 0.866 |
| Phosphatidylcholine (C18:2,C20:4) | 0.949 | 0.988 | 0.943 | 0.954 |
| Glyoxylate | 0.946 | 0.942 | 0.912 | 0.968 |
| Eicosenoic acid (C20:cis[11]1) | 0.943 | 1.421 | 1.163 | 0.818 |
| scyllo-Inositol | 0.934 | 1.179 | 1.032 | 0.875 |
| beta-Carotene | 0.933 | 1.086 | 0.980 | 0.903 |
| Lysophosphatidylcholine (C16:0) | 0.932 | 0.976 | 0.919 | 0.941 |
| Lysophosphatidylethanolami ne (C18:0) | 0.927 | 0.950 | 0.899 | 0.946 |
| Coenzyme Q9 | 0.925 | 0.967 | 0.907 | 0.937 |
| O-Phosphotyrosine | 0.919 | 1.063 | 0.950 | 0.893 |
| Lysophosphatidylcholine (C18:0) | 0.918 | 1.014 | 0.926 | 0.913 |
| Lysophosphatidylcholine (C18:2) | 0.912 | 1.044 | 0.935 | 0.896 |
| Phosphatidylcholine No 02 | 0.907 | 0.937 | 0.873 | 0.932 |
| Coenzyme Q10 | 0.897 | 0.896 | 0.842 | 0.939 |
| Eicosadienoic acid (C20:2) No 02 | 0.890 | 1.358 | 1.077 | 0.793 |
| Lactate | 0.884 | 1.090 | 0.929 | 0.852 |
| 2-Aminoadipinic acid | 0.869 | 1.493 | 1.108 | 0.742 |
| Phosphatidylcholine (C18:0,C20:4) | 0.856 | 0.897 | 0.803 | 0.896 |
| Phosphatidylcholine (C18:0,C22:6) | 0.846 | 0.909 | 0.799 | 0.879 |
| Dodecanol | 0.834 | 1.058 | 0.862 | 0.814 |
| Adenosine monophosphate (AMP) | 0.825 | 0.919 | 0.783 | 0.852 |
| Glucuronic acid | 0.718 | 0.788 | 0.626 | 0.794 |
| Gluconic acid | 0.712 | 0.833 | 0.636 | 0.764 |
| Maltotriose | 0.680 | 0.717 | 0.553 | 0.771 |

**Table 7: Metabolites that differentiate between different levels of tumor status pT in prostate cancer tissue. Estimated changes and p-values were calculated by a mixed-effect linear model (ANOVA with subject as random factor) on log10 transformed ratios. Criteria of classification are given in Table 9.**

| | pT Score (high versus low) |
|---|---|
| **Metabolite** | **Estimated change** |
| 2-Hydroxybehenic acid (C22:0) | 1.323 |
| Cerebronic acid (2-OH-C24:0) | 1.512 |
| Pentadecanol | 1.218 |
| Pseudouridine | 1.047 |
| myo-Inositol, lipid fraction | 1.043 |
| 14-Methylhexadecanoic acid | 1.309 |
| Eicosapentaenoic acid (C20:cis[5,8,11,14,17]5) | 1.244 |
| Arginine | 1.166 |
| Erythronic acid | 0.996 |
| Tricosanoic acid (C23:0) | 1.237 |
| myo-Inositol-2-phosphate, lipid fraction | 1.046 |
| 7-Methylguanine | 1.238 |
| Isopentenyl pyrophosphate (IPP) | 1.215 |
| erythro-C16-Sphingosine | 0.991 |
| Glycerophosphoethanolamine, polar fraction | 1.192 |
| Dihydrocholesterol | 1.374 |
| Cholestenol No 02 | 1.180 |
| threo-Sphingosine | 0.969 |
| Glycerol-2-phosphate | 1.157 |
| Pyruvate | 1.101 |
| Threonic acid | 1.227 |
| Docosahexaenoic acid (C22:cis[4,7,10,13,16,19]6) | 1.078 |
| Sphingosine isomer No 01 | 1.056 |
| 3-O-Methylsphingosine | 1.044 |
| erythro-Dihydrosphingosine | 0.995 |
| 5-O-Methylsphingosine | 1.021 |
| myo-Inositol-2-phosphate | 1.035 |
| erythro-Sphingosine | 1.016 |
| Nicotinamide | 0.996 |
| Cytosine | 1.010 |
| Flavine adenine dinucleotide (FAD) | 1.105 |
| Lignoceric acid (C24:0) | 1.122 |
| Nervonic acid (C24:cis[15]1) | 1.119 |
| DAG (C18:1,C18:2) | 1.077 |
| Sphingosine-1-phosphate | 1.074 |
| Behenic acid (C22:0) | 1.041 |
| Nicotinic acid | 0.901 |
| Phytosphingosine, total | 0.969 |
| Eicosanoic acid (C20:0) | 1.087 |
| dihomo-gamma-Linolenic acid (C20:cis[8,11,14]3) | 1.103 |
| Sedoheptulose-7-phosphate | 0.991 |
| Phosphocreatine | 1.080 |
| Glycolate | 0.952 |
| beta-Sitosterol | 0.922 |
| Sphingomyelin (d18:1,C24:0) | 0.940 |
| Sphingomyelin (d18;1,C16:0) | 0.943 |
| Phosphatidylcholine (C18:0,C18:1) | 0.941 |
| Ceramide (d18:1,C24:1) | 1.003 |
| Lysophosphatidylcholine (C20:4) | 1.043 |
| Phosphatidylcholine (C16:0,C20:4) | 0.940 |
| gamma-Tocopherol | 1.060 |
| Phosphatidylcholine (C18:2,C20:4) | 0.996 |
| Glyoxylate | 1.064 |
| Eicosenoic acid (C20:cis[11]1) | 1.103 |
| scyllo-Inositol | 0.959 |
| beta-Carotene | 1.190 |
| Lysophosphatidylcholine (C16:0) | 0.911 |
| Lysophosphatidylethanolamine (C18:0) | 0.925 |
| Coenzyme Q9 | 0.909 |
| O-Phosphotyrosine | 1.131 |
| Lysophosphatidylcholine (C18:0) | 0.959 |
| Lysophosphatidylcholine (C18:2) | 1.031 |
| Phosphatidylcholine No 02 | 0.938 |
| Coenzyme Q10 | 0.856 |
| Eicosadienoic acid (C20:2) No 02 | 1.133 |
| Lactate | 0.959 |
| 2-Aminoadipinic acid | 1.183 |
| Phosphatidylcholine (C18:0,C20:4) | 0.929 |
| Phosphatidylcholine (C18:0,C22:6) | 0.936 |
| Dodecanol | 0.961 |
| Adenosine monophosphate (AMP) | 0.936 |
| Glucuronic acid | 0.585 |
| Gluconic acid | 0.649 |
| Maltotriose | 0.686 |

**Table 8: Metabolites that differentiate between different levels of Gleason score in serum. Estimated changes and p-values were calculated by a linear model with age, BMI and sample storage time as fixed effects (ANOVA) on log10 transformed ratios. Criteria of classification are given in Table 9.**

| | Gleason Score Bi (high versus low) | Gleason Score Tri (high versus intermediate) | Gleason Score Tri (high versus low) | Gleason Score Tri (high versus low) |
|---|---|---|---|---|
| Metabolite | Estimated change | Estimated change | Estimated change | Estimated change |
| 3,4-Dihydroxyphenylalanine (DOPA) | 0.978 | 0.970 | 0.961 | 0.990 |
| Hexadecanol | 1.024 | 0.946 | 0.989 | 1.046 |
| Indole-3-lactic acid | 0.957 | 1.167 | 1.052 | 0.901 |
| Potassium | 0.970 | 0.951 | 0.940 | 0.988 |
| 3,4-Dihydroxyphenyl-acetic acid (DOPAC) | 1.059 | 1.066 | 1.101 | 1.032 |
| Adrenaline (Epinephrine) | 1.023 | 1.020 | 1.035 | 1.015 |
| Androstenedione | 1.157 | 0.968 | 1.134 | 1.173 |
| Corticosterone | 1.264 | 0.845 | 1.143 | 1.352 |
| Cortisol | 1.248 | 0.870 | 1.148 | 1.320 |
| 3,4-Dihydroxyphenylglycol (DOPEG) | 0.984 | 0.998 | 0.983 | 0.985 |
| 4-Hydroxy-3-methoxymandelic acid | 1.185 | 0.884 | 1.101 | 1.245 |
| Hentriacontane | 1.007 | 1.009 | 1.013 | 1.003 |
| Metanephrine | 0.960 | 1.166 | 1.053 | 0.993 |
| Galactitol | 1.040 | 0.995 | 1.037 | 1.042 |
| myo-Inositol-2-phosphate | 0.888 | 0.941 | 0.855 | 0.909 |
| Normetanephrine | 1.177 | 0.987 | 1.168 | 1.183 |
| 3-Indoxylsulfate | 1.134 | 0.813 | 0.999 | 1.228 |
| Glycochenodeoxycholic acid | 0.992 | 0.952 | 0.962 | 1.011 |
| Isopalmitic acid (C16:0) | 1.210 | 1.131 | 1.306 | 1.155 |
| Phosphatidylcholine (C16:0,C18:2) | 0.990 | 1.001 | 0.991 | 0.990 |
| Phosphatidylcholine (C18:1,C18:2) | 0.997 | 0.997 | 0.995 | 0.998 |
| Phosphatidylcholine (C16:1,C18:2) | 1.078 | 0.957 | 1.050 | 1.097 |
| Sphingomyelin (d18:1,C24:0) | 1.036 | 0.975 | 1.020 | 1.046 |
| Phosphatidylcholine (C18:0,C18:1) | 1.010 | 0.991 | 1.005 | 1.014 |
| Lysophosphatidyl-choline (C18:1) | 1.073 | 1.007 | 1.077 | 1.070 |
| beta-Sitosterol | 0.980 | 1.005 | 0.984 | 0.978 |
| beta-Carotene | 1.084 | 1.083 | 1.137 | 1.050 |
| Arginine | 1.037 | 0.957 | 1.009 | 1.055 |
| Threonic acid | 1.014 | 0.942 | 0.977 | 1.037 |
| Lactate | 0.974 | 0.984 | 0.964 | 0.980 |
| Pyruvate | 0.914 | 1.021 | 0.925 | 0.907 |
| Canthaxanthin | 0.842 | 1.236 | 0.957 | 0.774 |
| Cryptoxanthin | 0.848 | 0.925 | 0.809 | 0.875 |
| Lycopene | 0.952 | 1.087 | 1.001 | 0.921 |
| gamma-Tocopherol | 0.972 | 1.035 | 0.993 | 0.959 |
| Coenzyme Q10 | 0.910 | 0.872 | 0.838 | 0.961 |
| Eicosapentaenoic acid (C20:cis[5,8,11,14,17]5) | 1.049 | 0.889 | 0.975 | 1.097 |
| Docosahexaenoic acid (C22:cis[4,7,10,13,16,19]6) | 0.899 | 0.987 | 0.892 | 0.903 |
| Lignoceric acid (C24:0) | 1.087 | 0.978 | 1.073 | 1.097 |
| Behenic acid (C22:0) | 1.035 | 0.994 | 1.031 | 1.037 |
| Nervonic acid (C24:cis[15]1) | 0.962 | 1.067 | 1.002 | 0.939 |
| dihomo-gamma-Linolenic acid (C20:cis[8,11,14]3) | 0.991 | 1.044 | 1.018 | 0.975 |
| Pseudouridine | 0.986 | 0.960 | 0.962 | 1.002 |
| Dodecanol | 1.025 | 0.998 | 1.024 | 1.026 |
| Eicosanoic acid (C20:0) | 0.976 | 0.993 | 0.972 | 0.979 |
| Pentadecanol | 1.186 | 0.993 | 1.181 | 1.190 |
| gamma-Linolenic acid (C18:cis[6,9,12]3) | 1.021 | 0.872 | 0.939 | 1.076 |
| Tricosanoic acid (C23:0) | 1.131 | 1.120 | 1.213 | 1.083 |
| myo-Inositol-2-phosphate, lipid fraction | 0.793 | 1.143 | 0.861 | 0.754 |
| erythro-C16-Sphingosine | 1.023 | 1.079 | 1.072 | 0.994 |
| erythro-Dihydrosphingosine | 0.956 | 0.892 | 0.891 | 0.999 |
| erythro-Sphingosine | 1.009 | 1.034 | 1.030 | 0.997 |
| Dehydroepiandrosterone sulfate | 1.384 | 1.264 | 1.597 | 1.264 |
| scyllo-Inositol | 0.818 | 0.864 | 0.747 | 0.865 |
| 14-Methylhexadecanoic acid | 1.253 | 1.053 | 1.293 | 1.228 |
| Ketoleucine | 0.910 | 0.940 | 0.876 | 0.932 |
| Glucuronic acid | 0.953 | 1.081 | 0.999 | 0.924 |
| Testosterone | 0.948 | 0.796 | 0.827 | 1.040 |
| .conjugated Linoleic acid (C18:trans[9,11]2) | 1.141 | 1.081 | 1.197 | 1.107 |
| Erythronic acid | 1.052 | 0.907 | 0.991 | 1.092 |
| 1,5-Anhydrosorbitol | 1.057 | 1.006 | 1.061 | 1.054 |
| Dihydrotestosterone | 0.937 | 0.880 | 0.869 | 0.987 |
| 5-Hydroxy-3-indoleacetic acid (5-HIAA) | 1.072 | 0.954 | 1.042 | 1.092 |
| 4-Hydroxy-3-methoxyphenylglycol (HMPG) | 1.038 | 1.028 | 1.056 | 1.027 |
| 3-Methoxytyrosine | 1.001 | 0.886 | 0.931 | 1.051 |
| Phytosphingosine, total | 1.019 | 1.083 | 1.071 | 0.988 |
| DAG (C18:1,C18:2) | 0.977 | 0.961 | 0.954 | 0.992 |
| MetID(68300045) | 1.079 | 0.887 | 1.004 | 1.132 |
| Phosphatidylcholine (C16:0,C20:4) | 0.994 | 0.990 | 0.988 | 0.998 |
| Phosphatidylcholine (C18:0,C18:2) | 1.004 | 1.003 | 1.006 | 1.003 |
| Phosphatidylcholine (C18:0,C22:6) | 0.971 | 0.969 | 0.953 | 0.983 |
| MetID(68300048) | 1.030 | 0.982 | 1.018 | 1.037 |
| Lysophosphatidylcholine (C18:2) | 1.163 | 0.896 | 1.089 | 1.215 |
| Lysophosphatidylcholine (C20:4) | 1.049 | 0.970 | 1.030 | 1.062 |
| Phosphatidylcholi ne (C16:0,C16:0) | 1.030 | 1.044 | 1.057 | 1.012 |
| Phosphatidylcholine (C18:2,C20:4) | 0.989 | 1.017 | 0.999 | 0.982 |
| MetID(68300017) | 1.137 | 0.985 | 1.126 | 1.144 |
| Phosphatidylcholine No 02 | 1.004 | 1.002 | 1.005 | 1.004 |
| MetID(68300015) | 0.900 | 0.990 | 0.895 | 0.903 |
| Phosphatidylcholine (C18:0,C20.4) | 0.974 | 0.984 | 0.964 | 0.980 |
| MetID(68300020) | 1.012 | 0.969 | 0.993 | 1.025 |
| MetID(68300047) | 0.966 | 0.937 | 0.929 | 0.991 |
| Ceramide (d18:1,C24:0) | 0.974 | 1.056 | 1.007 | 0.953 |
| Ceramide (d18:1,C24:1) | 0.956 | 1.107 | 1.016 | 0.918 |
| Sphingomyelin (d18:1,C16:0) | 0.996 | 1.006 | 1.000 | 0.993 |
| Sphingomyelin (d18:1,C16:0) | 0.992 | 0.997 | 0.991 | 0.994 |
| Cholestenol No 02 | 0.979 | 1.057 | 1.013 | 0.958 |
| 3-O-Methylsphingosine | 1.005 | 1.065 | 1.045 | 0.981 |
| 5-O-Methylsphingosine | 1.007 | 1.053 | 1.040 | 0.987 |
| Lysophosphatidylcholine (C18:0) | 1.043 | 0.967 | 1.022 | 1.057 |
| Lysophosphatidylcholine (C16:0) | 1.034 | 0.935 | 0.993 | 1.063 |
| Lysophosphatidylcholine (C16:0) | 1.001 | 0.990 | 0.995 | 1.005 |
| Lysophosphatidylcholine (C17:0) | 1.191 | 1.027 | 1.211 | 1.178 |
| Choline plasmalogen (C18,C20:4) | 0.967 | 0.974 | 0.952 | 0.978 |

**Table 9: Criteria for classification of Gleason Score and pT Score in low, intermediate and high**

| | | |
|---|---|---|
| | low | Gleason score 2-6 |
| Gleason Score Bi | high | Gleason Score 7-10 |
| | low | Gleason Score 2-6 |
| | intermediate | Gleason Score 7 |
| Gleason Score Tri | high | Gleason Score 8-10 |
| | low | pT2 |
| pT Score | high | pT3-4 |

## Claims

1. A method for diagnosing a prostate carcinoma or a predisposition therefor comprising:
(a) determining the metabolite 7-Methylguanine in a test sample of a subject suspected to suffer from said prostate carcinoma or to have a predisposition therefore, and
(b) comparing the test results of the determination in step (a) to a reference, whereby said prostate carcinoma or a predisposition therefor is to be diagnosed.

2. The method of claim 1, wherein at least an additional metabolite is determined selected from the group consisting of: 2-Hydroxybehenic acid (C22:0), Cerebronic acid (2-OH-C24:0), Isopentenyl pyrophosphate (IPP), 14-Methylhexadecanoic acid, 2-Aminoadipinic acid, Ceramide (d18:1, C24:1), Eicosenoic acid (C20:cis[11]1), Tricosanoic acid (C23:0), Glycerophosphoethanolamine, Arginine, Behenic acid (C22:0), beta-Carotene, Cytosine, DAG (C18:1, C18:2), Dihydrocholesterol, Erythro-Dihydrosphingosine, Docosahexaenoic acid (C22:cis[4,7,10,13,16,19]6), Dodecanol, Eicosanoic acid (C20:0), Eicosapentaenoic acid (C20:cis[5,8,11,14,17]5), Dihomo-gamma-Linolenic acid (C20:cis[8,11,14]3), erythro-C16-Sphingosine, Flavine adenine dinucleotide (FAD), gamma-Tocopherol, Gluconic acid, Glucuronic acid, Glycerol-2-phosphate, Lignoceric acid (C24:0), Lysophosphatidylcholine (C18:2), Lysophosphatidylcholine (C20:4), Maltotriose; myo-Inositol; myo-Inositol-2-phosphate, Nervonic acid (C24:cis[15]1), Nicotinamide, Pentadecanol, Phosphatidylcholine (C18:0, C22:6), Phytosphingosine, Pseudouridine, Pyruvate, 3-O-Methylsphingosine, threo-Sphingosine, 5-O-Methylsphingosine, erythro-Sphingosine, Sphingosine-1-phosphate, Threonic acid, Choline plasmalogen (C18,C20:4), 2-Oxoisocaproic acid, metabolite belonging to the class of choline plasmalogens and exhibiting the following characteristic ionic species when detected with LC/MS, applying electro-spray ionization (ESI) mass spectrometry: mass-to-charge ratio (m/z) of the positively charged ionic species is 767 (+/- 0.5), metabolite belonging to the class of choline plasmalogens and exhibiting the following characteristic ionic species when detected with LC/MS, applying electro-spray ionization (ESI) mass spectrometry: mass-to-charge ratio (m/z) of the positively charged ionic species is 768.6 (+/- 0.5), Erythronic acid, myo-Inositolphospholipids, metabolite exhibiting the following characteristic ionic fragments when detected with GC/MS, applying electron impact (EI) ionization mass spectrometry, after acidic methanolysis and derivatisation with 2% O-methylhydroxylamine-hydrochlorid in pyridine and sub-sequently with N-methyl-N-trimethylsilyltrifluoracetamid: MS (EI, 70 eV): m/z (%): 73 (100), 375 (38), 147 (27), 217 (19), 257 (14), 376 (14), 169 (14), 75 (10), 250 (7), 133 (7), 1,5-Anhydrosorbitol, 3-Hydroxybutyrate, 3-Methoxytyrosine, 4-Hydroxy-3-methoxyphenylglycol (HMPG), 5-Hydroxy-3-indoleacetic acid (5-HIAA), beta-Sitosterol, Canthaxanthin, Ceramide (d18:1, C24:0), metabolite belonging to the class of choline plasmalogens and exhibiting the following characteristic ionic species when detected with LC/MS, applying electro-spray ionization (ESI) mass spectrometry: mass-to-charge ratio (m/z) of the positively charged ionic species is 772.6 (+/- 0.5), Coenzyme Q10, conjugated Linoleic acid (C18:trans[9,11]2), Cryptoxanthin, Dihydrotestosterone, gamma-Linolenic acid (C18:cis[6,9,12]3), Glycerate, Lactate, Lycopene, Lysophosphatidylcholine (16:0), Lysophosphatidylcholine (C17:0), Phosphatidylcholine (C16:0, C20:4), Phosphatidylcholine (C18:0, C18:2), Phosphatidylcholine (C18:2, C20:4), ,metabolite belonging to the class of glycerophosphatidylcholines and exhibiting the following characteristic ionic species when detected with LC/MS, applying electro-spray ionization (ESI) mass spectrometry: mass-to-charge ratio (m/z) of the positively charged ionic species is 780.8 (+/- 0.5), Phosphatidylcholine, metabolite belonging to the class of glycerophosphatidylcholines and exhibiting the following characteristic ionic species when detected with LC/MS, applying electro-spray ionization (ESI) mass spectrometry: mass-to-charge ratio (m/z) of the positively charged ionic species is 796.8 (+/- 0.5), scyllo-Inositol, Sphingomyelin (d18:1, C16:0), metabolite belonging to the class of diacylglycerides and exhibiting the following characteristic ionic species when detected with LC/MS, applying electro-spray ionization (ESI) mass spectrometry: mass-to-charge ratio (m/z) of the positively charged ionic species is 695.6 (+/- 0.5), Testosterone, Dehydroepiandrosterone sulfate Eicosadienoic acid (C20:2) isomer No 02 exhibiting the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 81 (100), 57 (98), 43 (92), 67 (85), 41 (80), 55 (74), 82 (66), 95 (64), 110 (39), 109 (39), Cholestenol isomer No 02 exhibiting the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 143 (100), 458 (91), 73 (68), 81 (62), 95 (36), 185 (23), 327 (23), 368 (20), 255 (15), 429 (15), and Sphingosine isomer No 01 exhibiting the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-Ntrimethylsilyltrifluoracetamid: MS (EI, 70 eV): m/z (%): 73 (100), 250 (37), 412 (37), 147 (21), 322 (14), 413 (11), 128 (9), 251 (7), 500 (2).

3. The method of claim 1 or 2, wherein said reference is derived from a subject known to suffer from a prostate carcinoma or a subject known to have a predisposition therefor.

4. The method of claim 3, wherein identical or similar results for the test sample and the reference are indicative for a prostate carcinoma or a predisposition therefor.

5. The method of claim 1, wherein said reference is derived from a subject known to not suffer from a prostate carcinoma or a subject known to have no predisposition therefor.

6. The method of claim 1, wherein said reference is a calculated reference for the said at least one metabolite in a population of subjects.

7. The method of claim 5 or 6, wherein the absence of the said at least one metabolite or an amount thereof which differs in the test sample in comparison to the reference sample is indicative for a prostate carcinoma or a predisposition therefor.

8. The method of any one of claims 1 to 7, wherein at least one additional metabolite is determined selected from the group consisting of: Biotin, Uridine, Hypoxanthine, Inosine, Glycine, Cysteine, Cystine, Uracil, Aspartate, Isoleucine, trans-4-Hydroxyproline, Proline, Methionine, Glycerol-3-phosphate, 5-Oxoproline, Folic acid, Glutamate, Glutamine, Leucine, Myristic acid (C14:0), Phenylalanine, Heptadecanoic acid (C17:0), Citrulline, Threonine, myo-Inositol-1-phosphate, myo-Inositolphospholipids, Ribose, Fumarate, Tryptophan, Glycerol, Tyrosine, Homoserine, Histidine, Linoleic acid (C18:cis[9,12]2), Xanthine, Ornithine, Arginine, Citrulline, Pantothenic acid, Palmitoleic acid (C16:cis[9]1), Succinate, Fructose, alpha-Tocopherol, Nicotineamide adenine dinucleotide (NAD), Maltose, Valine, Adenine, Lysine, Malate, Alanine, Spermidine, Palmitic acid (C16:0), Stearic acid (C18:0), Oleic acid (C18:cis[9]1), Glycerol phosphate, N-Acetylneuraminic acid, Xylitol, Serine, N-Acetylneuraminic acid, S-Adenosylmethionine, Phosphate, Glucose, Cholesterol, Spermine, Putrescine, cis-Aconitate, Citrate, Ribulose-5-phosphate, Pyrophosphate (PPi), Elaidic acid (C18:trans[9]1), Adenine, 2-Hydroxybutyrate, Sarcosine, Isocitrate, Creatine, Glutathione disulfide (GSSG), 3-Hydroxybutyrate, and Taurine.

9. The method of any one of claims 1 to 8, wherein said determining the said at least - one metabolite comprises mass spectrometry (MS).

10. The method of claim 9, wherein said mass spectrometry is liquid chromatography (LC) MS and/or gas chromatography (GC) MS.

11. The method of any one of claims 1 to 10, wherein said sample is a sample of a body fluid of said subject.

12. The method of any one of claims 1 to 10, wherein said subject is a human.

13. Use of the metabolite 7-Methylguanine or a compound which specifically detects said metabolite for diagnosing a prostate carcinoma or a predisposition therefor in a sample of a subject.

14. Use of claim 13, wherein the metabolite is used in combination with at least one additional metabolite or a compound which specifically detects said additional metabolite, wherein said additional metabolite is selected from the group consisting of: 2-Hydroxybehenic acid (C22:0), Cerebronic acid (2-OH-C24:0), Isopentenyl pyrophosphate (IPP), 14-Methylhexadecanoic acid, 2-Aminoadipinic acid, Ceramide (d18:1, C24:1), Eicosenoic acid (C20:cis[11]1), Tricosanoic acid (C23:0), Glycerophosphoethanolamine, Arginine, Behenic acid (C22:0), beta-Carotene, Cytosine, DAG (C18:1, C18:2), Dihydrocholesterol, Erythro-Dihydrosphingosine, Docosahexaenoic acid (C22:cis[4,7,10,13,16,19]6), Dodecanol, Eicosanoic acid (C20:0), Eicosapentaenoic acid (C20:cis[5,8,11,14,17]5), Dihomo-gamma-Linolenic acid (C20:cis[8,11,14]3), erythro-C16-Sphingosine, Flavine adenine dinucleotide (FAD), gamma-Tocopherol, Gluconic acid, Glucuronic acid, Glycerol-2-phosphate, Lignoceric acid (C24:0), Lysophosphatidylcholine (C18:2), Lysophosphatidylcholine (C20:4), Maltotriose, myo-Inositol, myo-Inositol-2-phosphate, Nervonic acid (C24:cis[15]1), Nicotinamide, Pentadecanol, Phosphatidylcholine (C18:0, C22:6), Phytosphingosine, Pseudouridine, Pyruvate, 3-O-Methylsphingosine, threo-Sphingosine, 5-O-Methylsphingosine, erythro-Sphingosine, Sphingosine-1-phosphate, Threonic acid, Choline plasmalogen (C18,C20:4), 2-Oxoisocaproic acid, metabolite belonging to the class of choline plasmalogens and exhibiting the following characteristic ionic species when detected with LC/MS, applying electro-spray ionization (ESI) mass spectrometry: mass-to-charge ratio (m/z) of the positively charged ionic species is 767 (+/- 0.5), metabolite belonging to the class of choline plasmalogens and exhibiting the following characteristic ionic species when detected with LC/MS, applying electro-spray ionization (ESI) mass spectrometry: mass-to-charge ratio (m/z) of the positively charged ionic species is 768.6 .+/- 0.5), Erythronic acid, myo-Inositolphospholipids, metabolite exhibiting the following characteristic ionic fragments when detected with GC/MS, applying electron impact (EI) ionization mass spectrometry, after acidic methanolysis and derivatisation with 2% Omethylhydroxylamine-hydrochlorid in pyridine and sub-sequently with N-methyl-N-trimethylsilyltrifluoracetamid: MS (EI, 70 eV): m/z (%): 73 (100), 375 (38), 147 (27), 217 (19), 257 (14), 376 (14), 169 (14), 75 (10), 250 (7), 133 (7), 1,5-Anhydrosorbitol,
3-Hydroxybutyrate, 3-Methoxytyrosine, 4-Hydroxy-3-methoxyphenylglycol (HMPG), 5-Hydroxy-3-indoleacetic acid (5-HIAA), beta Sitosterol, Canthaxanthin, Ceramide
(d18:1, C24:0), metabolite belonging to the class of choline plasmalogens and exhibit-in the following characteristic ionic species when detected with LC/MS, applying electro spray ionization (ESI) mass spectrometry: mass to charge ratio (m/z) of the
positively charged ionic species is 772.6 (+/- 0.5), Coenzyme Q10, conjugated Linoleic acid (C18:trans[9,11]2) , Cryptoxanthin, Dihydrotestosterone, gamma-Linolenic acid (C18:cis[6,9,12]3), Glycerate, Lactate, Lycopene, Lysophosphatidylcholine (16:0), Lysophosphatidylcholine (C17:0), Phosphatidylcholine (C16:0, C20:4), Phosphatidylcholine (C18:0, C18:2), Phosphatidylcholine (C18:2, C20:4), metabolite belonging to the class of glycerophosphatidylcholines and exhibiting the following characteristic ionic species when detected with LC/MS, applying electro-spray ionization (ESI) mass spectrometry: mass-to-charge ratio (m/z) of the positively charged ionic species is 780.8 (+/- 0.5), Phosphatidylcholine, metabolite belonging to the class of glycerophosphatidylcholines and exhibiting the following characteristic ionic species when detected with LC/MS, applying electro-spray ionization (ESI) mass spectrometry: mass-to-charge ratio (m/z) of the positively charged ionic species is 796.8 (+/- 0.5), scyllo-inositol, Sphingomyelin (d18:1, C16:0), metabolite belonging to the class of diacylglycerides and exhibiting the following characteristic ionic species when detected with LC/MS, applying electro-spray ionization (ESI) mass spectrometry: mass-to-charge ratio (m/z) of the positively charged ionic species is 695.6 (+/- 0.5), Testosterone, Dehydroepiandrosterone sulfate Eicosadienoic acid (C20:2) isomer No 02 exhibiting the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 81 (100), 57 (98), 43 (92), 67 (85), 41 (80), 55 (74), 82 (66), 95 (64), 110 (39), 109 (39), Cholestenol isomer No 02 exhibiting the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-N-trimethylsilyltrifluoracetamid: MS (EI, 70 eV): m/z (%): 143 (100), 458 (91), 73 (68), 81 (62), 95 (36), 185 (23), 327 (23), 368 (20), 255 (15), 429 (15), and Sphingosine isomer No 01 exhibiting the following characteristic ionic fragments when detected with GC/MS, applying electron impact (EI) ionization mass spectrometry, after acidic methanolysis and derivatisation with 2% O-methylhydroxylamine-hydrochlorid in pyridine and subsequently with N-methyl-Ntrimethylsilyltrifluoracetamid: MS (EI, 70 eV): m/z (%): 73 (100), 250 (37), 412 (37), 147 (21), 322 (14), 413 (11), 128 (9), 251 (7), 500 (2) .

## Patentansprüche

1. Verfahren zur Diagnostizierung eines Prostatakarzinoms oder einer Prädisposition dafür, das Folgendes umfasst:
(a) Bestimmen des Metaboliten 7-Methylguanin in einer Testprobe aus einem Subjekt, das in Verdacht steht, an dem Prostatakarzinom zu leiden oder eine Prädisposition dafür aufzuweisen, und
(b) Vergleichen der Testergebnisse der Bestimmung in Schritt (a) mit einer Referenz, wodurch das Prostatakarzinom oder eine Prädisposition dafür diagnostiziert werden soll.

2. Verfahren nach Anspruch 1, wobei mindestens ein zusätzlicher Metabolit bestimmt wird, der aus der Gruppe bestehend aus den Folgenden ausgewählt ist:
2-Hydroxybehensäure (C22:0), Cerebronsäure (2-OH-C24:0), Isopentenylpyrophosphat (IPP), 14-Methylhexadecansäure, 2-Aminoadipinsäure, Ceramid (d18:1, C24:1), Eicosensäure (C20:cis[11]1),
Tricosansäure (C23:0), Glycerophosphoethanolamin, Arginin, Behensäure (C22:0), beta-Carotin, Cytosin, DAG (C18:1, C18:2), Dihydrocholesterol, Erythro-Dihydrosphingosin, Docosahexaensäure (C22:cis[4,7,10,13,16,19]6), Dodecanol,
Eicosansäure (C20:0), Eicosapentaensäure (C20:cis[5,8,11,14,17]5), Dihomo-gamma-Linolensäure (C20:cis[8,11,14]3), erythro-C16-Sphingosin, Flavin-Adenin-Dinukleotid (FAD), gamma-Tocopherol, Gluconsäure, Glucuronsäure, Glycerol-2-phosphat,
Lignocerinsäure (C24:0), Lysophosphatidylcholin (C18:2), Lysophosphatidylcholin (C20:4), Maltotriose, myo-Inositol, myo-Inositol-2-phosphat, Nervonsäure (C24:cis[15]1), Nicotinamid, Pentadecanol, Phosphatidylcholin (C18:0, C22:6), Phytosphingosin, Pseudouridin, Pyruvat, 3-O-Methylsphingosin, threo-Sphingosin, 5-O-Methylsphingosin, erythro-Sphingosin, Sphingosin-1-phosphat, Threonsäure, Cholin-Plasmalogen (C18,C20:4), 2-Oxoisocapronsäure, Metabolit, der zu der Klasse der Cholinplasmalogene gehört und
bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 767 (+/-0,5), Metabolit, der zu der Klasse der Cholinplasmalogene gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 768,6 (+/-0,5), Erythronsäure, myo-Inositolphospholipide, Metabolit, der bei Detektion mit GC/MS unter Verwendung der Elektronenimpact(EI)-Ionisierungsmassen-spektrometrie, nach saurer Methanolyse und
Derivatisierung mit 2% O-Methylhydroxylamin-hydrochlorid in Pyridin und dann mit N-Methyl-N-trimethylsilylfluoracetamid die folgenden charakteristischen Ionenfragmente aufweist: MS (EI, 70 eV): m/z (%): 73 (100), 375 (38), 147 (27), 217 (19), 257 (14), 376 (14), 169 (14), 75 (10), 250 (7), 133 (7), 1,5-Anhydrosorbitol, 3-Hydroxybutyrat, 3-Methoxytyrosin, 4-Hydroxy-3-methoxyphenylglycol (HMPG), 5-Hydroxy-3-indol-essigsäure (5-HIAA), beta-Sitosterol, Canthaxanthin, Ceramid (d18:1, C24:0), Metabolit, der zu der Klasse der Cholinplasmalogene gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 772,6 (+/-0,5), Coenzym Q10, konjugierte Linolsäure (C18:trans[9,11]2), Cryptoxanthin, Dihydrotestosteron, gamma-Linolensäure (C18:cis-[6,9,12]3), Glycerat, Lactat, Lycopen, Lysophosphatidylcholin (16:0), Lysophosphatidylcholin (C17:0), Phosphatidylcholin (C16:0, C20:4),
Phosphatidylcholin (C18:0, C18:2), Phosphatidylcholin (C18:2, C20:4), Metabolit, der zu der Klasse der Glycerophosphatidylcholine gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 780,8 (+/-0,5), Phosphatidylcholin, Metabolit, der zu der Klasse der Glycerophosphatidylcholine gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 796,8 (+/-0,5), scyllo-Inositol, Sphingomyelin (d18:1, C16:0), Metabolit, der zu der Klasse der Diacylglyceriden gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 695,6 (+/-0,5), Testosteron, Dehydroepiandrosteronsulfat, Eicosadiensäure (C20:2) Isomer Nr. 02, das bei Detektion mit GC/MS unter Verwendung der Elektronenimpact(EI)-Ionisierungsmassenspektrometrie nach saurer Methanolyse und Derivatisierung mit 2% O-Methylhydroxylamin-hydrochlorid in Pyridin und dann mit N-Methyl-N-trimethylsilyltrifluoracetamid die folgenden charakteristischen Ionenfragmente aufweist: MS (EI, 70 eV): m/z (%): 81 (100), 57 (98), 43 (92), 67 (85), 41 (80), 55 (74), 82 (66), 95 (64), 110 (39), 109 (39), Cholestenol Isomer Nr. 02, das bei Detektion mit GC/MS unter Verwendung der Elektronenimpact(EI)-Ionisierungsmassenspektrometrie nach saurer Methanolyse und Derivatisierung mit 2% O-Methylhydroxylamin-hydrochlorid in Pyridin und dann mit N-Methyl-N-trimethylsilyltrifluoracetamid die folgenden charakteristischen Ionenfragmente aufweist: MS (EI, 70 eV): m/z (%): 143 (100), 458 (91), 73 (68), 81 (62), 95 (36), 185 (23), 327 (23), 368 (20), 255 (15), 429 (15), und Sphingosin Isomer Nr. 01, das bei Detektion mit GC/MS unter Verwendung der Elektronenimpact(EI)-Ionisierungsmassenspektrometrie nach saurer Methanolyse und Derivatisierung mit 2% O-Methylhydroxylamin-hydrochlorid in Pyridin und
dann mit N-Methyl-N-trimethylsilyltrifluoracetamid die folgenden charakteristischen Ionenfragmente aufweist: MS (EI, 70 eV) : m/z (%): 73 (100), 250 (37), 412 (37), 147 (21), 322 (14), 413 (11), 128 (9), 251 (7), 500 (2).

3. Verfahren nach Anspruch 1 oder 2, wobei die Referenz aus einem Subjekt, das bekanntermaßen an einem Prostatakarzinom leidet, oder einem Subjekt, das bekanntermaßen eine Prädisposition dafür aufweist, abgeleitet ist.

4. Verfahren nach Anspruch 3, wobei identische oder ähnliche Ergebnisse für die Testprobe und die Referenz ein Anzeichen für ein Prostatakarzinom oder eine Prädisposition dafür sind.

5. Verfahren nach Anspruch 1, wobei die Referenz aus einem Subjekt, das bekanntermaßen nicht an einem Prostatakarzinom leidet, oder einem Subjekt, das bekanntermaßen keine Prädisposition dafür aufweist, abgeleitet ist.

6. Verfahren nach Anspruch 1, wobei die Referenz eine berechnete Referenz für den mindestens einen Metaboliten in einer Population von Subjekten ist.

7. Verfahren nach Anspruch 5 oder 6, wobei die Abwesenheit des mindestens einen Metaboliten oder eine Menge desselben, welche in der Testprobe im Vergleich zur Referenzprobe abweicht, ein Anzeichen für ein Prostatakarzinom oder eine Prädisposition dafür ist.

8. Verfahren nach einem der Ansprüche 1 bis 7, wobei mindestens ein zusätzlicher Metabolit bestimmt wird, der aus der Gruppe, bestehend aus: Biotin,
Uridin, Hypoxanthin, Inosin, Glycin, Cystein, Cystin, Uracil, Aspartat, Isoleucin, trans-4-Hydroxyprolin, Prolin, Methionin, Glycerol-3-phosphat, 5-Oxoprolin, Folsäure, Glutamat, Glutamin, Leucin, Myristinsäure (C14:0), Phenylalanin, Heptadecansäure (C17:0), Citrullin, Threonin, myo-Inositol-1-phosphat, myo-Inositolphospholipiden, Ribose, Fumarat, Tryptophan, Glycerol, Tyrosin, Homoserin, Histidin, Linolsäure (C18:cis[9,12]2), Xanthin, Ornithin, Arginin, Citrullin, Pantothensäure, Palmitoleinsäure (C16:cis[9]1), Succinat, Fructose, alpha-Tocopherol, Nicotinamid-Adenin-Dinukleotid (NAD), Maltose, Valin, Adenin, Lysin, Malat, Alanin, Spermidin, Palmitinsäure (C16:0), Stearinsäure (C18:0), Ölsäure (C18:cis[9]1), Glycerolphosphat, N-Acetylneuraminsäure, Xylitol,
Serin, N-Acetylneuraminsäure, S-Adenosylmethionin, Phosphat, Glucose, Cholesterol, Spermin, Putrescin, cis-Aconitat, Citrat, Ribulose-5-phosphat, Pyrophosphat (PPi), Elaidinsäure (C18:trans[9]1), Adenin, 2-Hydroxybutyrat, Sarcosin, Isocitrat, Creatin, Glutathiondisulfid (GSSG), 3-Hydroxybutyrat und Taurin, ausgewählt ist.

9. Verfahren nach einem der Ansprüche 1 bis 8, wobei das Bestimmen des mindestens einen Metaboliten Massenspektrometrie (MS) umfasst.

10. Verfahren nach Anspruch 9, wobei es sich bei der Massenspektrometrie um Flüssigkeitschromatografie(LC)-MS und/oder um Gaschromatografie(GC)-MS handelt.

11. Verfahren nach einem der Ansprüche 1 bis 10, wobei es sich bei der Probe um eine Körperflüssigkeitsprobe des Subjekts handelt.

12. Verfahren nach einem der Ansprüche 1 bis 10, wobei es sich bei dem Subjekt um einen Menschen handelt.

13. Verwendung des Metaboliten 7-Methylguanin oder einer Verbindung, die den Metaboliten spezifisch nachweist, zur Diagnostizierung eines Prostatakarzinoms oder einer Prädisposition dafür in einer Probe aus einem Subjekt.

14. Verwendung nach Anspruch 13, wobei der Metabolit in Kombination mit mindestens einem zusätzlichen Metabolit oder einer Verbindung, die den zusätzlichen Metabolit spezifisch nachweist, verwendet wird, wobei der zusätzliche Metabolit aus der Gruppe bestehend aus den Folgenden ausgewählt ist: 2-Hydroxybehensäure (C22:0), Cerebronsäure (2-OH-C24:0), Isopentenylpyrophosphat (IPP), 14-Methylhexadecansäure, 2-Aminoadipinsäure, Ceramid (d18:1, C24:1), Eicosensäure (C20:cis[11]1), Tricosansäure (C23:0), Glycerophosphoethanolamin, Arginin, Behensäure (C22:0), beta-Carotin, Cytosin, DAG (C18:1, C18:2), Dihydrocholesterol, Erythro-Dihydrosphingosin, Docosahexaensäure (C22:cis[4,7,10,13,16,19]6), Dodecanol, Eicosansäure (C20:0), Eicosapentaensäure (C20:cis[5,8,11,14,17]5), Dihomo-gamma-Linolensäure (C20:cis[8,11,14]3), erythro-C16-Sphingosin, Flavin-Adenin-Dinukleotid (FAD), gamma-Tocopherol, Gluconsäure, Glucuronsäure, Glycerol-2-phosphat, Lignocerinsäure (C24:0), Lysophosphatidylcholin (C18:2), Lysophosphatidylcholin (C20:4), Maltotriose, myo-Inositol, myo-Inositol-2-phosphat, Nervonsäure (C24:cis[15]1), Nicotinamid, Pentadecanol, Phosphatidylcholin (C18:0, C22:6), Phytosphingosin, Pseudouridin, Pyruvat, 3-O-Methylsphingosin, threo-Sphingosin, 5-O-Methylsphingosin, erythro-Sphingosin, Sphingosin-1-phosphat, Threonsäure, Cholin-Plasmalogen (C18,C20:4), 2-Oxoisocapronsäure, Metabolit, der zu der Klasse der Cholinplasmalogene gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 767 (+/-0,5), Metabolit, der zu der Klasse der Cholinplasmalogene gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 768,6 (+/-0,5), Erythronsäure, myo-Inositolphospholipide, Metabolit, der bei Detektion mit GC/MS unter Verwendung der Elektronenimpact(EI)-Ionisierungsmassen-spektrometrie, nach saurer Methanolyse und Derivatisierung mit 2% O-Methylhydroxylamin-hydrochlorid in Pyridin und dann mit N-Methyl-N-trimethylsilylfluoracetamid die folgenden charakteristischen Ionenfragmente aufweist: MS (EI, 70 eV): m/z (%) : 73 (100), 375 (38), 147 (27), 217 (19), 257 (14), 376 (14), 169 (14), 75 (10), 250 (7), 133 (7), 1,5-Anhydrosorbitol, 3-Hydroxybutyrat, 3-Methoxytyrosin, 4-Hydroxy-3-methoxyphenylglycol (HMPG), 5-Hydroxy-3-indol-essigsäure (5-HIAA), beta-Sitosterol, Canthaxanthin, Ceramid (d18:1, C24:0), Metabolit, der zu der Klasse der Cholinplasmalogene gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 772,6 (+/-0,5), Coenzym Q10, konjugierte Linolsäure (C18:trans[9,11]2), Cryptoxanthin, Dihydrotestosteron, gamma-Linolensäure (C18:cis-[6,9,12]3), Glycerat, Lactat, Lycopen, Lysophosphatidylcholin (16:0), Lysophosphatidylcholin (C17:0), Phosphatidylcholin (C16:0, C20:4), Phosphatidylcholin (C18:0, C18:2), Phosphatidylcholin (C18:2, C20:4), Metabolit, der zu der Klasse der Glycerophosphatidylcholine gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 780,8 (+/-0,5), Phosphatidylcholin, Metabolit, der zu der Klasse der Glycerophosphatidylcholine gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 796,8 (+/-0,5), scyllo-Inositol, Sphingomyelin (d18:1, C16:0), Metabolit, der zu der Klasse der Diacylglyceriden gehört und bei Detektion mit LC/MS unter Verwendung der Elektrospray-Ionisierungs(ESI)-Massenspektrometrie die folgenden charakteristischen Ionenspezies aufweist: das Verhältnis von Masse zu Ladung (m/z) der positiv geladenen Ionenspezies beträgt 695,6 (+/-0,5), Testosteron, Dehydroepiandrosteronsulfat, Eicosadiensäure (C20:2) Isomer Nr. 02, das bei Detektion mit GC/MS unter Verwendung der Elektronenimpact(EI)-Ionisierungsmassenspektro-metrie nach saurer Methanolyse und Derivatisierung mit 2% O-Methylhydroxylamin-hydrochlorid in Pyridin und dann mit N-Methyl-N-trimethylsilyltrifluoracetamid die folgenden charakteristischen Ionenfragmente aufweist: MS (EI, 70 eV): m/z (%): 81 (100), 57 (98), 43 (92), 67 (85), 41 (80), 55 (74), 82 (66), 95 (64), 110 (39), 109 (39), Cholestenol Isomer Nr. 02, das bei Detektion mit GC/MS unter Verwendung der Elektronenimpact(EI)-Ionisierungsmassenspektrometrie nach saurer Methanolyse und Derivatisierung mit 2% O-Methylhydroxylamin-hydrochlorid in Pyridin und dann mit N-Methyl-N-trimethylsilyltrifluoracetamid die folgenden charakteristischen Ionenfragmente aufweist: MS (EI, 70 eV): m/z (%): 143 (100), 458 (91), 73 (68), 81 (62), 95 (36), 185 (23), 327 (23), 368 (20), 255 (15), 429 (15), und Sphingosin Isomer Nr. 01, das bei Detektion mit GC/MS unter Verwendung der Elektronenimpact(EI)-Ionisierungsmassenspektrometrie nach saurer Methanolyse und Derivatisierung mit 2% O-Methylhydroxylamin-hydrochlorid in Pyridin und dann mit N-Methyl-N-trimethylsilyltrifluoracetamid die folgenden charakteristischen Ionenfragmente aufweist: MS (EI, 70 eV): m/z (%): 73 (100), 250 (37), 412 (37), 147 (21), 322 (14), 413 (11), 128 (9), 251 (7), 500 (2).

## Revendications

1. Procédé pour diagnostiquer un carcinome de la prostate ou une prédisposition à celui-ci comprenant :
(a) la détermination du métabolite 7-méthylguanine dans un échantillon d'essai d'un sujet suspecté de souffrir dudit carcinome de la prostate ou d'avoir une prédisposition à celui-ci, et
(b) la comparaison des résultats d'essai de la détermination dans l'étape (a) à une référence, de sorte que ledit carcinome de la prostate ou une prédisposition à celui-ci soit diagnostiqué.

2. Procédé de la revendication 1, dans lequel au moins un métabolite additionnel est déterminé, choisi dans le groupe constitué de : l'acide 2-hydroxybéhénique (C22:0), l'acide cérébronique (2-OH-C24:0), le pyrophosphate d'isopentényle (IPP), l'acide 14-méthylhexadécanoïque, l'acide 2-aminoadipinique, le céramide (d18:1, C24:1), l'acide eicosénoïque (C20:cis[11]1), l'acide tri-cosanoïque (C23:0), la glycérophosphoéthanolamine, l'arginine, l'acide béhénique (C22:0), le bêta-carotène, la cytosine, DAG (C18:1, C18:2), le dihydrocholestérol, l'érythrodihydrosphingosine, l'acide docosahexaénoïque (C22:cis[4,7,10,13,16,19]6), le dodécanol, l'acide eicosanoïque (C20:0), l'acide eicosapentaénoïque (C20:cis[5,8,11,14,17]5), l'acide dihomo-gamma-linolénique (C20:cis[8,11,14)3), l'érythro-C16-sphingosine, le flavine adénine dinucléotide (FAD), le gamma-tocophérol, l'acide gluconique, l'acide glucuronique, le glycérol-2-phosphate, l'acide lignocérique (C24:0), la lysophosphatidylcholine (C18:2), la lysophosphatidylcholine (C20:4), le maltotriose, le myo-inositol, le myo-inositol-2-phosphate, l'acide nervonique (C24:cis[15]1), le nicotinamide, le pentadécanol, la phosphatidylcholine (C18:0, C22:6), la phytosphingosine, la pseudouridine, le pyruvate, la 3-O-méthylsphingosine, la thréosphingosine, la 5-O-méthylsphingosine, l'érythrosphingosine, le sphingosine-1-phosphate, l'acide thréonique, le plasmalogène de choline (C18,C20:4), l'acide 2-oxo-isocaproïque, un métabolite appartenant à la classe des plasmalogènes de choline et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est 767 (+/- 0,5), le métabolite appartenant à la classe des plasmalogènes de choline et présentant l'espèce ionique caractéristique suivante lorsqu'il est détecté par LC/MS, en appliquant la spectrométrie de masse par ionisation electrospray (ESI) : rapport masse/charge (m/z) de l'espèce ionique positivement chargée est 768,6 (+/-0,5), l'acide érythronique, des myo-inositolphospholipides, un métabolite présentant les fragments ioniques caractéristiques suivants lorsqu'il est détecté par GC/MS, en appliquant la spectrométrie de masse par ionisation par impact électronique (EI), après méthanolyse acide et dérivation avec 2 % de chlorhydrate de O-méthylhydroxylamine dans de la pyridine et ensuite avec du N-méthyl-N-triméthylsilyltrifluoracétamide : MS (EI, 70 eV) : m/z (%) : 73 (100), 375 (38), 147 (27), 217 (19), 257 (14), 376 (14), 169 (14), 75 (10), 250 (7), 133 (7), le 1,5-anhydrosorbitol, le 3-hydroxybutyrate, la 3-méthoxytyrosine, le 4-hydroxy-3-méthoxyphénylglycol (HMPG), l'acide 5-hydroxy-3-indoleacétique (5-HIAA), le bêta-sitostérol, la canthaxanthine, le céramide (d18:1, C24:0), un métabolite appartenant à la classe des plasmalogènes de choline et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est de 772,6 (+/-0,5), le coenzyme Q10, l'acide linoléique conjugué (C18:trans[9,11)2), la cryptoxanthine, la dihydrotestostérone, l'acide gamma-linolénique (C18:cis[6,9,12]3), le glycérate, le lactate, le lycopène, la lysophosphatidylcholine (16:0), la lysophosphatidylcholine (C17:0), la phosphatidylcholine (C16:0, C20:4), la phosphatidylcholine (C18:0, C18:2), la phosphatidylcholine (C18:2, C20:4), un métabolite appartenant à la classe des glycérophosphatidylcholines et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est de 780,8 (+/-0,5), la phosphatidylcholine, un métabolite appartenant à la classe des glycérophosphatidylcholines et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est 796,8 (+/-0,5), le scyllo-inositol, la sphingomyéline (d18:1, C16:0), un métabolite appartenant à la classe des diacylglycérides et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est 695,6 (+/-0,5), la testostérone, le sulfate de déshydroépiandrostérone, l'isomère d'acide eicosadiénoïque (C20:2) n° 2 présentant les fragments ioniques caractéristiques suivants lorsqu'ils sont détectés par GC/MS, en appliquant la spectrométrie de masse par ionisation par impact électronique (EI), après méthanolyse acide et dérivation avec 2 % de chlorhydrate de O-méthylhydroxylamine dans de la pyridine et ensuite avec du N-méthyl-N-triméthylsilyltrifluoracétamide : MS (EI, 70 eV) : m/z (%) : 81 (100), 57 (98), 43 (92), 67 (85), 41 (80), 55 (74), 82 (66), 95 (64), 110 (39), 109 (39), l'isomère de cholestérol n° 02 présentant les fragments ioniques caractéristiques suivants lorsqu'il est détecté par GC/MS, en appliquant la spectrométrie de masse par ionisation par impact électronique (EI), après méthanolyse acide et dérivation avec 2 % de chlorhydrate de O-méthylhydroxylamine dans de la pyridine et ensuite avec du N-méthyl-N-triméthylsilyltrifluoracétamide : MS. (EI, 70 eV) : m/z (%) : 143 (100), 458 (91), 73 (68), 81 (62), 95 (36), 185 (23), 327 (23), 368 (20), 255 (15), 429 (15), et l'isomère de sphingosine n° 01 présentant les fragments ioniques caractéristiques suivants lorsqu'il est détecté par GC/MS, en appliquant la spectrométrie de masse par ionisation par impact électronique (EI), après méthanolyse acide et dérivation avec 2 % de chlorhydrate de O-méthylhydroxylamine dans de la pyridine et ensuite avec du N-méthyl-N-triméthylsilyltrifluoracétamide : MS (EI, 70 eV) : m/z (%) : 73 (100), 250 (37), 412 (37), 147 (21), 322 (14), 413 (11), 128 (9), 251 (7), 500 (2).

3. Procédé de la revendication 1 ou 2, dans lequel ladite référence est dérivée d'un sujet connu pour souffrir d'un carcinome de la prostate ou un sujet connu pour avoir une prédisposition à celui-ci.

4. Procédé de la revendication 3, dans lequel des résultats similaires ou identiques pour l'échantillon d'essai et la référence sont indicatifs d'un carcinome de la prostate ou un prédisposition à celui-ci.

5. Procédé de la revendication 1, dans lequel ladite référence est dérivée d'un sujet connu pour ne pas souffrir d'un carcinome de la prostate ou d'un sujet connu pour avoir une prédisposition à celui-ci.

6. Procédé de la revendication 1, dans lequel ladite référence est une référence calculée pour ledit au moins un métabolite dans une population de sujets.

7. Procédé de la revendication 5 ou 6, dans lequel l'absence dudit au moins un métabolite ou une quantité de celui-ci qui diffère dans l'échantillon d'essai en comparaison à l'échantillon de référence est indicative d'un carcinome de la prostate ou d'une prédisposition à celui-ci.

8. Procédé de l'une quelconque des revendications 1 à 7, dans lequel au moins un métabolite additionnel est déterminé comme étant choisi dans le groupe constitué de : la biotine, l'uridine, l'hypoxanthine, l'inosine, la glycine, la cystéine, la cystine, l'uracile, l'aspartate, l'isoleucine, la trans-4-hydroxyproline, la proline, la méthionine, le glycérol-3-phosphate, la 5-oxoproline, l'acide folique, le glutamate, la glutamine, la leucine, l'acide myristique (C14:0), la phénylalanine, l'acide heptadécanoïque (C17:0), la citrulline, la thréonine, le myo-inositol-1-phosphate, les myo-inositolphospholipides, le ribose, le fumarate, le tryptophane, le glycérol, la tyrosine, l'homosérine, l'histidine, l'acide linoléique (C18:cis[9,12]2), la xanthine, l'ornithine, l'arginine, la citrulline, l'acide pantothénique, l'acide palmitoléique (C16:cis[9]1), le succinate, le fructose, l'alphatocophérol, le nicotinamide adénine dinucléotide (NAD), le maltose, la valine, l'adénine, la lysine, le malate, l'alanine, la spermidine, l'acide palmitique (C16:0), l'acide stéarique (c18:0), l'acide oléique (C18:cis[9]1), le phosphate de glycérol, l'acide N-acétylneuraminique, le xylitol, la sérine, l'acide N-acétylneuraminique, la S-adénosylméthionine, le phosphate, le glucose, le cholestérol, la spermine, la putrescine, le cis-aconitate, le citrate, le ribulose-5-phosphate, le pyrophosphate (PPi), l'acide élaïdique (C18:trans[9]1), l'adénine, le 2-hydroxybutyrate, la sarcosine, l'isocitrate, la créatine, le disulfure de glutathion (GSSG), le 3-hydroxybutyrate, et la taurine.

9. Procédé de l'une quelconque des revendications 1 à 8, dans lequel ladite détermination dudit au moins un métabolite comprenant la spectrométrique de masse (MS).

10. Procédé de la revendication 9, dans lequel ladite spectrométrie de masse est la chromatographie liquide (LC)-MS et/ou la chromatographie gazeuse (GC)-MS.

11. Procédé de l'une quelconque des revendications 1 à 10, dans lequel ledit échantillon est un échantillon d'un fluide corporel dudit sujet.

12. Procédé de l'une quelconque des revendications 1 à 10, dans lequel ledit sujet est un humain.

13. Utilisation du métabolite 7-méthylguanine ou un composé qui détecte spécifiquement ledit métabolite pour diagnostiquer un carcinome de la prostate ou une prédisposition à celui-ci dans un échantillon d'un sujet.

14. Utilisation de la revendication 13, dans laquelle le métabolite est utilisé en combinaison avec au moins un métabolite additionnel ou un composé qui détecte spécifiquement ledit métabolite additionnel, ledit métabolite additionnel étant choisi dans le groupe constitué de : l'acide 2-hydroxybéhénique (C22:0), l'acide cérébronique (2-OH-C24:0), le pyrophosphate d'isopentényle (IPP), l'acide 14-méthylhexadécanoïque, l'acide 2-aminoadipinique, le céramide (d18:1, C24:1), l'acide eicosénoïque (C20:cis[11]1), l'acide tri-cosanoïque (C23:0), la glycérophosphoéthanolamine, l'arginine, l'acide béhénique (C22:0), le bêta-carotène, la cytosine, DAG (C18:1, C18:2), le dihydrocholestérol, l'érythrodihydrosphingosine, l'acide docosahexaénoïque (C22:cis[4,7,10,13,16,19]6), le dodécanol, l'acide eicosanoïque (C20:0), l'acide eicosapentaénoïque (C20:cis[5,8,11,14,17]5), l'acide dihomo-gamma-linolénique (C20:cis[8,11,14)3), l'érythro-C16-sphingosine, le flavine adénine dinucléotide (FAD), le gamma-tocophérol, l'acide gluconique, l'acide glucuronique, le glycérol-2-phosphate, l'acide lignocérique (C24:0), la lysophosphatidylcholine (C18:2), la lysophosphatidylcholine (C20:4), le maltotriose, le myo-inositol, le myo-inositol-2-phosphate, l'acide nervonique (C24:cis[15]1), le nicotinamide, le pentadécanol, la phosphatidylcholine (C18:0, C22:6), la phytosphingosine, la pseudouridine, le pyruvate, la 3-O-méthylsphingosine, la thréosphingosine, la 5-O-méthylsphingosine, l'érythrosphingosine, le sphingosine-1-phosphate, l'acide thréonique, le plasmalogène de choline (C18,C20:4), l'acide 2-oxo-isocaproïque, un métabolite appartenant à la classe des plasmalogènes de choline et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est 767 (+/- 0,5), le métabolite appartenant à la classe des plasmalogènes de choline et présentant l'espèce ionique caractéristique suivante lorsqu'il est détecté par LC/MS, en appliquant la spectrométrie de masse par ionisation electrospray (ESI) : rapport masse/charge (m/z) de l'espèce ionique positivement chargée est 768,6 (+/-0,5), l'acide érythronique, des myo-inositolphospholipides, un métabolite présentant les fragments ioniques caractéristiques suivants lorsqu'il est détecté par GC/MS, en appliquant la spectrométrie de masse par ionisation par impact électronique (EI), après méthanolyse acide et dérivation avec 2 % de chlorhydrate de O-méthylhydroxylamine dans de la pyridine et ensuite avec du N-méthyl-N-triméthylsilyltrifluoracétamide : MS (EI, 70 eV) : m/z (%) : 73 (100), 375 (38), 147 (27), 217 (19), 257 (14), 376 (14), 169 (14), 75 (10), 250 (7), 133 (7), le 1,5-anhydrosorbitol, le 3-hydroxybutyrate, la 3-méthoxytyrosine, le 4-hydroxy-3-méthoxyphénylglycol (HMPG), l'acide 5-hydroxy-3-indoleacétique (5-HIAA), le bêta-sitostérol, la canthaxanthine, le céramide (d18:1, C24:0), un métabolite appartenant à la classe des plasmalogènes de choline et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est de 772,6 (+/-0,5), le coenzyme Q10, l'acide linoléique conjugué (C18:trans[9,11)2), la cryptoxanthine, la dihydrotestostérone, l'acide gamma-linolénique (C18:cis[6,9,12]3), le glycérate, le lactate, le lycopène, la lysophosphatidylcholine (16:0), la lysophosphatidylcholine (C17:0), la phosphatidylcholine (C16:0, C20:4), la phosphatidylcholine (C18:0, C18:2), la phosphatidylcholine (C18:2, C20:4), un métabolite appartenant à la classe des glycérophosphatidylcholines et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est de 780,8 (+/-0,5), la phosphatidylcholine, un métabolite appartenant à la classe des glycérophosphatidylcholines et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est 796,8 (+/-0,5), le scyllo-inositol, la sphingomyéline (d18:1, C16:0), un métabolite appartenant à la classe des diacylglycérides et présentant l'espèce ionique caractéristique suivante lorsqu'il est détecté par LC/MS, en appliquant la spectrométrie de masse par ionisation electrospray (ESI) : le rapport masse/charge (m/z) de l'espèce ionique positivement chargée est 695,6 (+/-0,5), la testostérone, le sulfate de déshydroépiandrostérone, l'isomère d'acide eicosadiénoïque (C20:2) n° 2 présentant les fragments ioniques caractéristiques suivants lorsqu'ils sont détectés par GC/MS, en appliquant la spectrométrie de masse par ionisation par impact électronique (EI), après méthanolyse acide et dérivation avec 2 % de chlorhydrate de O-méthylhydroxylamine dans de la pyridine et ensuite avec du N-méthyl-N-triméthylsilyltrifluoracétamide : MS (EI, 70 eV) : m/z (%) : 81 (100), 57 (98), 43 (92), 67 (85), 41 (80), 55 (74), 82 (66), 95 (64), 110 (39), 109 (39), l'isomère de cholestérol n° 02 présentant les fragments ioniques caractéristiques suivants lorsqu'il est détecté par GC/MS, en appliquant la spectrométrie de masse par ionisation par impact électronique (EI), après méthanolyse acide et dérivation avec 2 % de chlorhydrate de O-méthylhydroxylamine dans de la pyridine et ensuite avec du N-méthyl-N-triméthylsilyltrifluoracétamide : MS (EI, 70 eV) : m/z (%) : 143 (100), 458 (91), 73 (68), 81 (62), 95 (36), 185 (23), 327 (23), 368 (20), 255 (15), 429 (15), et l'isomère de sphingosine n° 01 présentant les fragments ioniques caractéristiques suivants lorsqu'il est détecté par GC/MS, en appliquant la spectrométrie de masse par ionisation par impact électronique (EI), après méthanolyse acide et dérivation avec 2 % de chlorhydrate de O-méthylhydroxylamine dans de la pyridine et ensuite avec du N-méthyl-N-triméthylsilyltrifluoracétamide : MS (EI, 70 eV) : m/z (%) : 73 (100), 250 (37), 412 (37), 147 (21), 322 (14), 413 (11), 128 (9), 251 (7), 500 (2).
